# EUROPÄISCHE PATENTSCHRIFT

(11) **EP 1 257 888 B1**
(45) Veröffentlichungstag und Bekanntmachung des Hinweises auf die Patenterteilung: **19.07.2006**
(21) Anmeldenummer: 01943197.2
(22) Anmeldetag: 22.01.2001
(51) Int. Cl.: G05F 1/42, A61L 9/22, F24F 3/16, C01B 13/11, H01T 23/00, B01J 19/08, G01N 33/00

(54) **VERFAHREN UND VORRICHTUNG ZUR REGELUNG DER KAPAZITÄT ZWISCHEN ZWEI ELEKTRODEN IN EINEM GAS**
METHOD AND DEVICE FOR CONTROLLING THE CAPACITY BETWEEN TWO ELECTRODES IN A GAS
PROCEDE ET DISPOSITIF POUR REGLER LA CAPACITE ENTRE DEUX ELECTRODES DANS UN GAZ

(30) Priorität: 01.02.2000 DE 10004326; 21.03.2000 DE 10013841
(43) Veröffentlichungstag der Anmeldung: 20.11.2002
(73) Patentinhaber: Automotive AG, 9490 Vaduz (LI)
(72) Erfinder: RUMP, Hanns, 63840 Hausen (DE); KIESEWETTER, Olaf, 98716 Geschwenda (DE)
(74) Vertreter: Mierswa, Klaus
(86) Internationale Anmeldenummer: PCT/EP2001/000672
(87) Internationale Veröffentlichungsnummer: WO 2001/062306

(56) Entgegenhaltungen:
- WO-A-85/01704
- WO-A-96/35115
- WO-A-97/03746
- WO-A-98/19962
- WO-A-98/26482
- DE-C- 4 217 621
- RU-C- 2 108 283
- US-A- 4 680 694
- US-A- 4 682 266
- US-A- 5 540 898

## Beschreibung

### Technisches Gebiet:

Die Erfindung betrifft ein Verfahren zur Regelung der elektrischen Kapazität, welche zwischen zwei Elektroden eines Gasentladungsmoduls besteht, wobei das Gasentladungsmodul eine Vorrichtung zur Erzeugung eines Plasmas zur Herstellung von Ozon und/oder Sauerstoffionen in Luft nach dem Prinzip der dielektrisch behinderten Entladung enthält und zwischen den Elektroden eine von einem steuerbaren Hochspannungsgenerator erzeugte Hochspannung angelegt ist, die zur Auslösung einer Gasentladung in dem Gas des Gasentladungsmoduls dient und die entweder eine Wechselspannung oder eine gepulste Gleichspannung ist, und die Gasentladung ein Plasma erzeugt, durch welches die effektive Fläche des Elektroden und damit die zwischen denselben bestehende Kapazität vergrößert wird, gemäß dem Oberbegriff des Anspruchs 1 sowie eine Vorrichtung gemäß dem Oberbegriff des Anspruchs 31.

### Stand der Technik:

Es ist bekannt, mit Hilfe von Ozon bzw. von aktiven Sauerstoffionen in lufttechnischen Anlagen Gase, Dampfe, üble Gerüche sowie Keime aller Art zu zerstören. Es ist bekannt, mit Hilfe von Gasentladungsmodulen Ozon bzw. aktive Sauerstoff-lonen nach der Methode der dielektrisch behinderten Entladung zu erzeugen mit dem Ziel, in der Luft enthaltene Gerüche und Keime oxidativ abzubauen. In DE 199 31 366.0 sind geeignete, sehr flache und kleine Gasentladungsmodule beschrieben.

Die bekannteste Bauform eines Gasentladungsmoduls zur Erzeugung von Ionen und Ozon ist die sogenannte Siemens-Röhre, die seit 1857 eingesetzt wird. Dabei sind in einer Glasröhre sowohl innen als auch außen zumeist aus Drahtgeflecht bestehende Elektroden-Netze angebracht. Bei Anlegen einer hohen Wechselspannung kommt es zu Entladungsvorgängen und kurzzeitigem Zünden von lokalen Plasmen, welche Ozon etc. erzeugen.

Der Wirkungsgrad derartiger Anlagen wird nach DE 199 33 180.4 durch den Einsatz eines Sorbtionskatalysators erhöht, welcher strömungstechnisch nach den lonisatoren angebracht ist. Auf den Oberflächen des Sorbtionskatalysators werden sowohl oxidierbare Luftbestandteile als auch Bakterien, sowie aktive Sauerstoffionen und Ozon aufkonzentriert. Damit wird jene kritische Konzentration beider Fraktionen erreicht, welche eine chemische Reaktion der beiden Fraktionen erst möglich macht. Vorteilhaft ist, daß überschüssiger Ozon vom Sorbtionskatalysator zerstört wird, sodaß kein Ozon an die Umgebung abgegeben wird.

Es ist in der DE 196 51 402.9 eine flache Baugruppe beschrieben worden, bei der sich auf beiden Seiten eines keramischen Dielektrikums und durch mehrere Schichten weiterer Dielektrika getrennt, geeignete Elektroden befinden, wobei die Rückseite durchgehend eine flächige Elektrode aufweist und die Vorderseite eine oder mehrere schmale, bandförmige Elektroden hat. Bei Anlegen einer hohen Wechselspannung ergeben sich ausgehend von den Bandelektroden auf der Vorderseite der flachen Baugruppe flächige Plasma-Leuchterscheinungen und es wird in großer Menge Ozon erzeugt.

Nachteilig bei allen bekannten Ausführungsformen von Gasentladungsmodulen ist, daß das bei zu hoher elektrischer Spannung entstehende überschüssige Ozon sehr unerwünscht ist und daß bei zu geringer elektrischer Spannung der Wirkungsgrad unzureichend ist. Problematisch ist, daß sich die Produktionsmenge von Ozon den unterschiedlichsten Beaufschlagungen der Luft mit Geruchsstoffen anpassen muß, weil stets ein bestimmtes Verhältnis von Ozon zu den luftgetragenen, oxidierbaren Luftbestandteilen bestehen muß. Wird zuwenig Ozon produziert, ist die Wirkung der Anlage unzureichend. Wird zuviel Ozon produziert, kann Ozon überschießen und belästigend wirken. Außerdem entstehen bei zu hoher elektrischer Spannung als Nebenprodukt stark ätzend wirkende und gesundheitsschädliche Stickoxide, welche die luftreinigende Wirkung dieser Geräte geradezu ad absurdum führen.

So wurde nachgewiesen, daß das Verhältnis von produziertem Ozon zu produzierten Stickoxiden bei üblichen Ionisationsrnodulen, z.B. von klassischen Siemensröhren, zumeist etwa 10 Teile Ozon und 1 Teil Stickoxid ist (10:1). Bei richtiger Auswahl der Dimensionierungen des Gasentladungsmoduls und bei dessen konstantem Betrieb in einem eng definierten Arbeitsbereich läßt sich dieses Verhältnis auf Werte von ca. 50:1 verbessern.

Dies ist für Anwendungen in lufttechnischen Anlagen deshalb von Bedeutung, weil Ozon als solcher in hohen Konzentrationen unerwünscht und durchaus gesundheitsrelevant ist, aber in sogenannten "natürlichen Konzentrationen" von ca. 50-200ppb (also Konzentrationen, wie sie z.B. in den Bergen oder an der See auch natürlich vorkommen) ohne negative Empfindungen und ohne weiteres physiologisch akzeptiert wird. Wird diesem "reinen Ozon" jedoch Stickoxid beigemischt, ändert sich sofort die physiologische Akzeptanz. Denn mit Stickoxid verunreinigter Ozon führt schon bei Konzentrationen von deutlich unter 1OOppb zu heftigen Reizungen am Bindegewebe, vor allem an Augen, Hals und Nase. Es ist daher wichtig, daß die lonisationsmodule so aufgebaut werden und elektrisch so angesteuert werden, daß der Anteil von Stickoxiden an der Ozonproduktion so klein wie möglich ist.

Nachteilig in vorgenanntem Sinne ist bei herkömmlichen lonisationsgeräten, daß ihre Wirkung, z.B. das Verhältnis von produzierter Ozonmenge zu produzierter Stickoxid-Menge, durch zahlreiche Faktoren stark beeinflußt wird. So muß z.B. bei Austausch des Gasentladungsmoduls aufgrund von bauartbedingten Toleranzen, Exemplarstreuungen jeweils die Größe der Hochspannung durch einen Eingriff von außen neu eingestellt werden muß und die Wirkung, also die Ozonproduktion und ggf. die Stickoxidproduktion, neu eingemessen werden müssen. Derartige Maßnahmen sind des Weiteren auch nötig, um Alterungseffekte des Gasentladungsmoduls, z.B. wegen Abbrand der Elektroden, sowie Änderungen des Zustandes, der Zusammensetzung, der relativen Feuchte und der Strömungsgeschwindigkeit des Gases zu auszugleichen.

Um diese Nachteile zu kompensieren, sind sensorgesteuerte Anlagen bekannt, die den Betrieb der Anlagen in einem definierten, günstigen Bereich geregelt konstant halten. Bekannt ist, in Strömungsrichtung hinter dem Gasentladungsmodul einen Ozonsensor vorzusehen, dessen Signal über einen Regler dem Hochspannungserzeuger so zugeführt wird, daß eine steigende Ozonproduktion eine verminderte elektrische Spannung zur Folge hat, was im Ergebnis zu einem gleichmäßigen und niemals zu hohem Ozonpegel führt. Nachteilig ist, daß genaue und langzeitstabile Ozonmeßeräte sehr teuer sind und preiswerte Sensoren in den geforderten Konzentrationsbereichen von weniger als 50ppb unzuverlässig sind.

Weiter wird im Stand der Technik vorgeschlagen, in Strömungsrichtung vor dem Gasentladungsmodul einen geeigneten Sensor anzuordnen, welcher die Strömungsgeschwindigkeit, die Luftfeuchte und die Konzentrationen an oxidierbaren Luftbestandteilen feststellt. Ein Steuergerät wertet diese Sensorsignale so aus, daß die lonisationsleistung dann erhöht wird, wenn
- die Luftfeuchte zunimmt,
- die Schadstoff-Konzentration in der Luft zunimmt,
- die Luftmenge bzw. die Strömungsgeschwindigkeit zunimmt bzw. umgekehrt. Diese Methoden sind aufwendig und erzeugen hohe Kosten.

Es ist weiter bekannt, daß oxidische Halbleitersensoren ihre elektrischen Parameter dann verändern, wenn oxidierbare oder reduzierbare Gase auf die Wirkschicht des Sensors einwirken und daß mit den so ermittelten Signalen derartiger Sensoren klima- und lüftungstechnische Anlagen sinnvoll und situationsabhängig zu steuern sind.

In PCT/EP84/00286 ist eine Sensorik beschrieben, welche die Lüftung eines Automobils derartig steuert, daß immer dann, wenn das Fahrzeug in eine Zone erhöhter Luftbelastung einfährt, die Lüftung von Normalbetrieb auf Umluftbetrieb umgeschaltet wird, und damit die hochbelastete Luft nicht die in Fahrzeugkabine hineinläßt.

In PCT/EP96/01609 ist eine lonisations-Anordung zur Anwendung in Automobilen beschrieben, bei der die lonisationsmodule im Luftstrom vor den Wärmetauschern (Heizung, Kühlung) und nach der Umluftklappe angeordnet sind. Erfindungsgemäß wird dadurch erreicht, daß einerseits oxidierbare Luftbestandteile, Gerüche, Keime, Gase, aufoxidiert und insofern vernichtet werden, und daß andererseits die auf der feuchten Oberfläche des Verdampfers, Kühlers, siedelnden Keime und Pilze wirkungsvoll vernichtet werden.

In der WO 01/02291 A2 ist eine Vorrichtung zur Erzeugung eines Plasmas zur Herstellung von Ozon und/oder Sauerstoffionen in Luft beschrieben, bei welcher sich zwischen zwei Elektroden mindestens ein Dielektrikum befindet, wobei eine Gasentladung nach dem Prinzip der dielektrisch behinderten Entladung erfolgt.

Aus der DE 42 17 621 C1 ist eine Vorrichtung zur Ozonerzeugung mit einem Ozonerzeuger, einem Gleichrichter, einem kapazitiven Zwischenkreis und einem Wechselrichter bekannt. An dem Ozonerzeuger ist eine Wechselspannung angelegt, deren Pulsform mit Hilfe einer Formschaltung veränderbar ist. Der Effektivwert der Wechselspannung kann unter Vorgabe eines Sollwertes mittels Pulsweiten-Modulation in einem ersten Regelkreis geregelt werden. Der Eingangswert für diesen ersten Regelkreis wird von einem Spannungsdetektor geliefert, welcher die am Ozonerzeuger anliegende Spannung mißt. Einem zweiten Regelkreis für die Wechselspannung wird der durch einen Ozonsensor ermittelte Ist-Wert der Ozonproduktion zugeführt.

Aus der WO 98/26482 A ist eine Vorrichtung zur Erzeugung aktiver Sauerstoffionen in der Luft für die Luftverbesserung, insbesondere von Atemluft, bekannt. Die Vorrichtung bestehet aus wenigstens einem Luftionisator und einem eine zur Luftionisierung ausreichende elektrische Hochspannung erzeugenden elektrischen Transformator. Der Luftionisator ist mit einem Luftgütesensor gekoppelt, welcher den Gehalt der Luft an oxidierbaren Gasen feststellt, wobei aufgrund des ermittelten Gehaltes derartiger oxidierbare Gase die dem Luftionisator zugeführte elektrische Energie mittels einer elektrischen Steuereinrichtung derart veränderbar ist, daß bei niedrigen Konzentrationen oxidierbarer Gase eine nur geringe Ionisationsleistung erfolgt, welche sensorgesteuert und automatisch mit zunehmender Konzentrationen an oxidierbaren Gasen auf einen Maximumwert steigerbar ist.

Aus der US 5 540 898 ist ein Ozonerzeuger bekannt, welcher mittels einer Gasentladung zwischen Elektroden aus reinem Sauerstoff Ozon erzeugt. Der reine Sauerstoff strömt aus einem Vorratsbehälter in ein die Elektroden enthaltendes Gehäuse. Der elektrische Entladestrom wird gemessen und zur Berechnung der erzeugten Ozonmenge herangezogen, welche durch Veränderung der an den Elektroden anliegenden Hochspannung oder durch Veränderung der in das Gehäuse einströmenden Sauerstoffmenge beeinflußbar ist.

Aus der US 4 682 266 ist eine Thyristorschaltung zur Spannungsversorgung einer Korona-Entladungsvorrichtung, z.B. Ozongenerator, bekannt. Diese Spannungsversorgung liefert einen Entladestrom, welcher durch eine zyklische Abfolge von positiven Rechteck-Impulsen, negativen Rechteck-Impulsen und Pausen gegeben ist. Die positiven und negativen Stromimpulse werden erzeugt, indem eine Gleichspannung in einem bestimmten Rhythmus zerhackt und der resultierende Strom fortlaufend umgepolt wird. Die Pulsweiten sowie die Pulshöhen sind dabei einstellbar. Der Strom wird hierbei gemäß einer Ausführungsform der Thyristorschaltung durch eine Regelung stabil gehalten.

Aus der WO 98 19 962 ist eine Spannungsversorgung für eine Korona-Gasentladungseinheit mit einem zwischen den Elektroden befindlichen Dielektrikum bekannt. Ein Zähler zählt die Spannungsdurchschläge durch das Dielektrikum und reduziert die zwischen den Elektroden anliegende Hochspannung, falls in einem Zeitintervall mehr als eine vorgegebene Anzahl von Spannungsdurchschlägen festgestellt wird, um das Dielektrikum gegen vorzeitige Alterung zu schützen und somit die Lebensdauer der Gasentladungseinheit zu verlängern.

Aus der WO 96 35115 ist ein Apparat zur Steuerung der Belüftung von Kabinen, insbesondere Fahrzeugen, bekannt, bei welchem Sensorewn zur Detektion von Diesel- und Benzinabgasen eingesetzt werden. Die Signale der Sensoren werden bei sich verschlechternder der Außenluftqualität zur Umschaltung von Zuluftbetrieb auf Umluftbetrieb herangezogen. Die Sensoren sind so geschaltet, daß die bei der Detektion von Diesel- oder Benzinabgasen ausgelöste Änderung des Sensorwiderstandes zu einer elektrischen Signalveränderung führt, die unabhängig von der Natur des Gases den gleichen Richtungsverlauf hat.

Aus der RU 2 108 283 C1 ist ein System zur Herstellung ozonisierter Luft bekannt. Das System umfaßt eine Spannungsversorgung, einen Hochspannungsgenerator, einen Pulsgenerator, eine programmierte Pulsweiten- und Pulsfrequenzsteuerung sowie einen Lüfter.

Aus der US 4 680 694 ist eine weitere Thyristorschaltung zur Spannungsversorgung eines Ozongenerators bekannt. Ein Teil der Schaltung bildet einen Resonanzkreis mit eine bestimmten Resonanzfrequenz. Die Thyristoren werden mit einer geringeren Frequenz geschaltet als die Resonanzfrequenz mit dem Ergebnis, daß die von der Thyristorschaltung abgegebene Spannung im wesentlichen gepulst ist.

Aus der WO 85 01 704 A1 sind ein Verfahren und eine Vorrichtung zur Reinigung von schadstoffbelasteter Luft bekannt. Hierbei wird die zu reinigenden Luft zunächst teilweise erwärmt und dann einem Chemisorptionsfilter nachgeschalteten Katalysatormassen zugeführt. Aus der WO 97 037 46 A1 schließlich ist ein Verfahren zur plasmatischen Zersetzung und/oder Vernichtung von Schadstoffen bekannt. Die Schadstoffe durchlaufen eine mit dielektrisch behinderten Entladungen beaufschlagte Reaktorstrecke, welche Entladungzonen und entladungsfreie Zonen aufweist.

### Technische Aufgabe:

Der Erfindung liegt die Aufgabe zu Grunde, ein Verfahren zum Betreiben eines Gasentladungsmoduls bereitzustellen, mit welchem die genannten Nachteile vermieden werden. Der Erfindung liegt ferner die Aufgabe zu Grunde, eine Vorrichtung bereitzustellen, welche unter Vermeidung der oben genannten Nachteile dennoch eine Gasentladung mit dauerhaft konstanter Wirkung ermöglicht. Der Erfindung liegt des Weiteren die Aufgabe zu Grunde, ein Verfahren zum Betreiben eines Gasentladungsmoduls bereitzustellen, mit welchem eine automatische Anpassung der Gasentladung an den Grad der Belastung des Gases mit Fremdstoffen, Schadstoffen oder Keimen ermöglicht. Der Erfindung liegt des Weiteren die Aufgabe zu Grunde, ein Verfahren bereitzustellen, welches die Feststellung des Erschöpfungsgrades eines Filters erlaubt.

### Offenbarung der Erfindung sowie deren Vorteile:

Diese Aufgabe wird erfindungsgemäß gelöst durch ein Verfahren zur Regelung der elektrischen Kapazität, welche zwischen zwei Elektroden eines Gasentladungsmoduls besteht, wobei das Gasentladungsmodul eine Vorrichtung zur Erzeugung eines Plasmas zur Herstellung von Ozon und/oder Sauerstoffionen in Luft nach dem Prinzip der dielektrisch behinderten Entladung enthält und zwischen den Elektroden eine von einem steuerbaren Hochspannungsgenerator erzeugte Hochspannung angelegt ist, die zur Auslösung einer Gasentladung in dem Gas des Gasentladungsmoduls dient und die entweder eine Wechselspannung oder eine gepulste Gleichspannung ist, und die Gasentladung ein Plasma erzeugt, durch welches die effektive Fläche der Elektroden und damit die zwischen denselben bestehende Kapazität vergrößert wird, dadurch gekennzeichnet, daß, wenn der Ist-Wert der zwischen den beiden Elektroden bestehenden Kapazität unterhalb eines bestimmten Sollwertes liegt, der Effektivwert der Hochspannung des Hochspannungsgenerators zeitlich zunehmend gesteuert wird, oder wenn der Ist-Wert der zwischen den beiden Elektroden bestehenden elektrischen Kapazität oberhalb des Sollwertes liegt, der Effektivwert der Hochspannung des Hochspannungsgenerators zeitlich abnehmend gesteuert wird.

Die Aufgabe wird ferner gelöst durch eine Vorrichtung zur Erzeugung einer Gasentladung, umfassend ein Gasentladungsmodul mit zwei in einem Gas, insbesondere Luft, befindlichen Elektroden, und einen steuerbaren Hochspannungsgenerator, welcher entweder eine Wechselspannung oder eine gepulste Gleichspannung abgibt, wobei das Gasentladungsmodul eine Vorrichtung zur Erzeugung eines Plasmas zur Herstellung von Ozon und/oder Sauerstoffionen in Luft nach dem Prinzip der dielektrisch behinderten Entladung enthält, und die Elektroden so an den Hochspannungsgenerator angeschlossen sind, daß die Wechselspannung oder die gepulste Gleichspannung zwischen den Elektroden anliegt, und die Gasentladung ein Plasma erzeugt, durch welches die effektive Fläche der Elektroden und damit die zwischen denselben bestehende Kapazität vergrößert wird, dadurch gekennzeichnet,
daß die Vorrichtung ferner einen Regler umfaßt, welcher den Ist-Wert der zwischen den beiden Elektroden bestehenden Kapazität oder einen hieraus abgeleiteten Wert mit einem vorgebbaren Sollwert vergleicht,
wobei, wenn der Ist-Wert der zwischen den beiden Elektroden bestehenden Kapazität unterhalb eines bestimmten Sollwertes liegt, der Effektivwert der Hochspannung des Hochspannungsgenerators durch den Regler zeitlich zunehmend gesteuert wird,
oder wenn der Ist-Wert der zwischen den beiden Elektroden bestehenden elektrischen Kapazität oberhalb des Sollwertes liegt, der Effektivwert der Hochspannung des Hochspannungsgenerators durch den Regler zeitlich abnehmend gesteuert wird.

Ein Ziel der Erfindung ist es, mit Hilfe der beim Betrieb von elektrischen lonisationsmodulen bzw. Gasentladungsmodulen nach dem Prinzip der dielektrisch behinderten Entladung entstehenden elektrischen Effekte Regelgrößen zu gewinnen, diese dem Hochspannungsgenerator zuzuführen und insofern einen geschlossenen Regelkreis zu bilden mit dem Ergebnis, daß der Arbeitsbereich des lonisationsmoduls stets konstant bleibt.

Elektrische Gasentladungsmodule aller Art, welche zumeist nach dem Prinzip der dielektrisch behinderten Entladung arbeiten, erzeugen durch energiereiche elektrische Entladungsstoß aktive Sauerstoffionen, Ozon und oxidieren damit alle Luftanteile. Neben der nützlichen Eigenschaft des oxidativen Zerstörens von Bakterien, Viren, Pilzen und oxidierbaren Gasen, Dämpfen und den meisten Gerüchen wird der in der Luft enthaltene Stickstoff dann oxidiert, wenn die lonisationsmodule aufgrund der elektrischen Ansteuerung Entladungsimpulse erzeugen, deren Energieniveau ausreicht, um Stickstoff N₂ zu 2xN₁ zu dissoziieren, welcher sich dann mit dem naszierenden Sauerstoff O₁ zu NO und anschließend zu NO₂ verbindet.

Dieser unerwünschte Prozess findet insbesondere dann statt, wenn die in das Gasentladungsmodul eingespeiste Spannung einen zu hohen Wert annimmt. Daraus folgt, daß die Spannung am Gasentladungsmodul stets auf einem bestimmten Wert gehalten wird, welcher einerseits genug Ozon produziert, andererseits noch kein Stickoxid produziert.

Die Erfindung beschreibt Methoden, welche elektrisch auswertbare Effekte im Zusammenhang mit dem Betrieb von Gasentladungsmodulen auswertet und aus diesen Effekten Regelgrößen ableitet mit dem Ziel, das Gasentladungsmodul stets und konstant in einem definierten Arbeitspunkt zu betreiben, welcher eine besonders große Spreizung zwischen der Produktion von Ozon und Stickoxid aufweist.

Die Erfindung ermöglicht es, unter Ausnutzung rein elektrischer Parameter ein Gasentladungsmodul so anzusteuern, daß sich eine erhebliche Vergleichmäßigung der Arbeitspunkte ergibt, ohne daß aufwendige und kostentreibende zusätzliche Sensoren benötigt werden. Vorteilhaft erfolgt durch die Lehre der Erfindung die Produktion von Ozon und von aktiven Sauerstoffionen in einem Bereich, in welchem die Produktion unerwünschter Nebenprodukte wie NOx noch nicht erfolgt. Auf diese Weise kann der Anteil von Stickoxiden bei der Ozonproduktion stets und unabhängig von den genannten Parametern sehr niedrig gehalten werden.

Hierbei erfolgt erfindungsgemäß eine automatische Anpassung an die Luftfeuchte, die Temperatur, den Druck und die Geschwindigkeit der anströmenden Luft, d.h., die Auswirkung von Änderungen dieser Parameter auf die Wirkung des Gasentladungsmoduls werden erfindungsgemäß automatisch kompensiert. Dabei nutzt die Erfindung zum Zwecke der Gewinnung von Ozon von eine Regelung ermöglichenden Informationen rein elektrische und vergleichsweise einfach und kostengünstig zu gewinnende Daten aus, so daß vorteilhafterweise keiner der genannten Parameter durch einen Sensor erfaßt werden muß.

Das Gasentladungsmodul kann mit Wechselspannung oder mit gepulster Gleichspannung betrieben werden, wobei die Wellenform erfindungsgemäß sinusförmig oder im wesentlichen rechteckförmig sein kann. Die Zeitkonstante der Regelung kann dabei kürzer sein als die halbe Periode der Wechselspannung oder der gepulsten Gleichspannung, so daß durch die Regelung die Wellenform der Wechselspannung oder der gepulsten Gleichspannung beeinflußt wird. Die Zeitkonstante kann dabei so kurz gewählt werden, daß ein zunächst sinusförmiger Verlauf der Wellenform im Scheitelbereich der Sinuswelle gestaucht wird und sich bei weiter abnehmender Zeitkonstante immer mehr einem rechteckigen Verlauf annähert. Die Frequenz der Wechselspannung oder der gepulsten Gleichspannung liegt typischerweise zwischen 15 kHz und 35 kHz. Die ideale Betriebsspannung liegt bei typisch ca. 4200 - 4800 Volt.

In einer bevorzugten Ausgestaltung der Erfindung befindet sich zwischen den beiden Elektroden mindestens ein Dielektrikum. Eine Vorrichtung zur Erzeugung eines Plasmas zur Herstellung von Ozon und/ oder Sauerstoffionen in Luft nach dem Prinzip der dielektrisch behinderten Entladung ist in der WO 01 / 02291 A2 beschrieben und umfaßt einen flachen, elektrisch isolierenden Träger, dessen Material eine Dielektrizitätskonstante von wenigstens größer 30 aufweist. Auf der einen Hauptoberfläche, Rückseite, des Trägers ist eine Elektrode aus einem elektrisch leitfähigen Material aufgebracht, auf der anderen, der Luft ausgesetzten Hauptoberfläche, Vorderseite, des Trägers, ist wenigstens eine elektrische Isolierschicht aus einem dielektrischen Material aufgebracht, wobei die Isolierschicht die Vorderseite des Trägers nur teilweise bedeckt: Die Dielektrizitätskonstante des Trägers und diejenige der Isolierschicht sind unterschiedlich, wobei die Differenz zwischen der Dielektrizitätskonstante des Trägers und der Isolierschicht bzw. der Teilschichten so gewählt ist, dass sich der Effekt der Spiegelentladungen einstellt, auf der Isolierschicht ist ebenfalls eine Elektrode, aus einem elektrisch leitfähigen Material angeordnet, welche die Isolierschicht nur teilweise bedeckt. An die beiden Elektroden ist die Hochspannung des Hochspannungsgenerators gelegt.

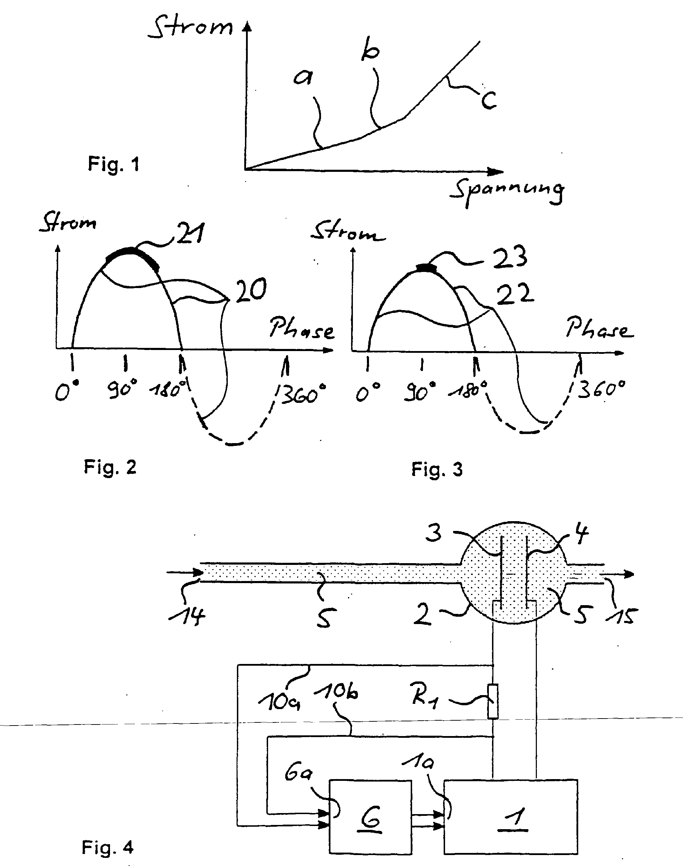

Die Figuren 1-3 dienen zur Erläuterung des der Erfindung zu Grunde liegenden Prinzips. In Fig. 1 ist der in einem Gasentladungsmodul fließende, durch die hohen Wechselspannung in Gang gesetzte Strom in Abhängigkeit von der Spannung schematisch dargestellt. Diese Abhängigkeit ist nicht - wie etwa bei einem ohmschen Widerstand - linear, sonder nichtlinear. Das Abknicken der Kurve von Fig. 1 begründet sich daher, daß bis zum Beginn der Steigungsänderung die speisende Wechselspannungsquelle nur durch den Scheinwiderstand Xc des als elektrischer Kondensator wirkenden lonisationsmoduls belastet wird, was eine streng lineare Funktion ist. Solange nämlich die Größe der Wechselspannung nicht ausreicht, ein Plasma zu erzeugen und damit eine Gasentladung in Gang zu setzen, bilden die Elektroden des Gasentladungsmoduls einen Kondensator mit einer festen Kapazität, welche sich aus den Abmessungen und der gegenseitigen Anordnung der Elektroden ergibt und im folgenden statische Kapazität C₀ genannt wird. Die Wechselspannungsquelle wird nur durch den - frequenzabhängigen - kapazitiven Widerstand dieser Kapazität belastet, so daß sich eine streng lineare Beziehung zwischen Strom und Spannung ergibt (Kurvenabschnitt a in Fig. 1). Die statische Kapazität zwischen den Elektroden beträgt typischerweise ca. 20-30pF.

Wenn jedoch die Zündspannung erreicht ist und sich an den Oberflächen der Elektroden des Gasentladungsmoduls ein Plasma ausbildet, wird durch dieses leitfähige Plasma die funktionale Fläche des Kondensators erhöht und es kommt zu einer plötzlichen Verringerung des Scheinwiderstandes Xc mit der Folge einer Stromerhöhung. Die Bildung des Plasmas bewirkt also eine Vergrößerung der effektiven als Elektrode wirkenden Fläche. Mit der Bildung des Plasmas geht daher eine Vergrößerung der elektrischen Kapazität, welche zwischen den Elektroden besteht, einher.

Da das Gasentladungsmodul als elektrische Kapazität zusammen mit der Induktivität des die Hochspannung liefernden Transformators einen L-C-Kreis darstellt, erfährt bei Zuschaltung der erhöhten Kapazität die Phasenlage des Stromes in Bezug auf die Spannung eine deutliche Änderung, die die erfolgte Zündung des Plasmas signalisiert.

Wenn die Zündspannung erreicht oder überschritten ist, bilden sich bei Gasentladungsmodulen nach dem Prinzip der dielektrisch behinderten Entladung kurze aber energiereiche Entladungsstöße aus, die kurz nach dem Aufflammen wieder erlöschen und sich von der Zeit und vom Ort des Auftretens her stochastisch verhalten. Jeder Entladungsstoß erzeugt ein energiereiches Plasma, welches als Leuchterscheinung gut zu beobachten ist und Filament genannt wird. Es bilden sich unaufhörlich neue Entladungen in sehr großer Anzahl, die kurze Zeit später wieder verlöschen. Die zwischen den Elektroden bestehende Kapazität sind daher schnellen Fluktuationen unterworfen. Der zwischen den Elektroden bestehende kapazitive Widerstand und daher auch der durch die Hochspannung in Gang gesetzte Strom fluktuieren daher ebenfalls, so daß sich ein typisches Frequenzspektrum bildet, welches als Rauschen den Strom durch die Anordnung überlagert.

Die Dauer der einzelnen Entladungen ist eine Funktion des Dielektrikums und der Geometrie der Anordnung. Typisch kann die Brennzeit eines Filamentes mit ca. 0,1-1 psek benannt werden, so daß sich ein Rauschspektrum ergibt mit einer Mittenfrequenz von ca. 2-5MHz.

Fig. 2 zeigt die Abhängigkeit des durch eine sinusförmige Hochspannung in Gang gesetzten Stromes von der Zeit und die Entstehung des elektrischen Rauschens bei einer ersten Größe der Wechselspannung. Diese ist in Fig. 2 so gewählt, daß nur in einem Bereich um den Scheitel der Sinuswelle eine Zündung des Plasmas erfolgt und nur die Halbwelle (Phase 0°-180° der Sinuswelle) gezeigt ist. Das Zünden des Plasmas macht sich durch eine Zunahme des Stromes bemerkbar, wobei diese Zunahme aufgrund der schnellen Fluktuationen der zwischen den Elektroden bestehenden Kapazität ebenfalls schnellen Fluktuationen unterworfen ist und das oben erläuterte elektrische Rauschen verursacht, das sich dem sinusförmigen Verlauf überlagert (in Fig. 2 und Fig. 3 als schwarze Fläche oberhalb des Scheitelbereiches der ersten Halbwelle dargestellt).

Es ist erkennbar, daß bei Spannungen unterhalb der Zündspannung sich ein reiner Sinus abbildet, ohne Überlagerungen durch Rauscherscheinungen. Im Bereich des Scheitelpunktes, Phase 90°, der Sinuswelle zünden in dichter zeitlicher Abfolge die Filamente bei einer Phase von ca. 70° und brennen bis zu einer Phase von ca.130°.

Bei einer bestimmten zweiten Größe der Wechselspannung (Fig. 3), welche ein wunschgemäß energiereiches Plasma erzeugt, wird beobachtet, daß das Plasma bei einem Winkel von ca. 88° erstmalig zündet und bis zu einem Winkel von ca. 110° brennt. Es wurde weiter beobachtet, daß sich bei konstanter Spannung sowohl die Lage des Auftreten des Rauschens auf dem Spannungs-Sinus als auch die Phasenlage des Stromes (in Bezug auf die angelegte Spannung) bei Verschiebung des Arbeitspunktes ändert. Die Lage des ersten Zündzeitpunktes des Plasmas auf dem Spannungs-Sinus wird durch Einfluß von Luftfeuchte, Anströmgeschwindigkeit und Anteile oxidierbarer gas- und dampfförmiger Luftbestandteile beeinflußt. Es wurde weiter beobachtet, daß das Rauschspektrum und die Rauschamplitude über die beiden Spitzenwerte einer Sinusperiode nicht identisch sind. Vielmehr treten mehr Filamente dann auf, wenn auf der Bandelektrode an der Vorderseite des lonisators die positive Halbwelle ansteht. Steht die negative Halbwelle an, bilden sich weitaus weniger Filamente. Ferner wird beobachtet, daß sich das Spektrum der durch Filamente gebildeten Rauschspannung ändert, wenn eine sehr hohe Spannung angelegt wird, so daß bei ansteigendem Sinus , weit vor Erreichen des Scheitelpunktes (90°) schon z.B. 60° die Zündspannung erreicht ist. Die Filamentproduktion verläuft sodann bei zunehmender Spannung bis zum Scheitelpunkt der Sinuswelle mit zunehmender Intensität, um dann wieder abzunehmen und bei ca. 140° vollständig zu erlöschen. Daß die Spannung beim Erlöschen der FilamentProduktion geringer ist als die erste Zündspannung, erklärt sich dadurch, daß auf der absteigenden Flanke der Sinuswelle die Luft in unmittelbarer Nähe der lonisationsstrecken bzw. Gasentladungsstrecken noch stark ionisiert ist, was die Zündfähigkeit für eine elektrische Entladung begünstigt.

Der durch die Hochspannung in Gang gesetzte elektrische Strom wächst mit der zwischen den Elektroden bestehenden elektrischen Kapazität an. Der Ist-Wert der zwischen den beiden Elektroden bestehenden elektrischen Kapazität kann daher aus der Größe des durch die Hochspannung in Gang gesetzten elektrischen Stromes bestimmt oder abgeleitet werden. Zu diesem Zweck kann ein Stromsensor den durch die Hochspannung in Gang gesetzten elektrischen Strom erfassen und in ein erstes Sensorsignal umsetzen und der Ist-Wert der zwischen den beiden Elektroden bestehenden elektrischen Kapazität aus dem ersten Sensorsignal bestimmt werden. Z.B. kann der elektrische Strom bestimmt werden, indem er über einen ersten Widerstand geführt und die an diesem abfallende Spannung abgegriffen und als Maß für den durch die Hochspannung in Gang gesetzten elektrischen Strom herangezogen wird. In diesem Fall fungiert der erste Widerstand als Stromsensor, wobei die dort abgegriffene Spannung als erstes Sensorsignal dient.

Der Ist-Wert der zwischen den beiden Elektroden bestehenden elektrischen Kapazität kann ferner aus
- der integralen Intensität oder
- Intensität mindestens einer bestimmten Frequenz oder mindestens eines bestimmten Frequenzbereiches oder
- der mittleren Frequenz oder
- der Frequenzverteilung oder
- der Frequenz der maximalen Intensität
des statistischen elektrischen Rauschens, mit welchem der durch die Hochspannung in Gang gesetzte elektrische Strom aufgrund der Gasentladung behaftet ist, bestimmt oder abgeleitet werden, da diese Parameter mit der zwischen den beiden Elektroden bestehenden elektrischen Kapazität korrelieren.

Z.B. kann ein Rauschsensor das statistische elektrische Rauschen, mit welchem der durch die Hochspannung in Gang gesetzte Strom aufgrund der Gasentladung behaftet ist, erfassen und in ein zweites Sensorsignal umsetzen und der Ist-Wert der zwischen den beiden Elektroden bestehenden elektrischen Kapazität aus dem zweiten Sensorsignal bestimmt werden

Das statistische elektrische Rauschen des Stromes kann erfaßt werden, indem der Strom über einen zweiten Widerstand oder eine Induktivität geführt wird, von welchem bzw. welcher das statistische elektrische Rauschen über ein als Bandpaß oder als Hochpaß fungierendes frequenzselektives elektrisches Filter abgegriffen wird. In diesem Fall umfaßt also der Rauschsensor einen zweiten Widerstand oder eine Induktivität, über den bzw. über die der durch die Hochspannung in Gang gesetzte elektrische Strom geführt ist, und ein als Bandpaß oder als Hochpaß fungierendes frequenzselektives elektrisches Filter, wobei die an dem zweiten Widerstand bzw. an der Induktivität abfallende Spannung abgegriffen wird und nach Passieren des frequenzselektiven elektrischen Filters als zweites Sensorsignal dient.

Der Ist-Wert der zwischen den beiden Elektroden bestehenden elektrischen Kapazität kann ferner aus
- der integralen Intensität oder
- Intensität mindestens einer bestimmten Frequenz oder mindestens eines bestimmten Frequenzbereiches oder
- der mittleren Frequenz oder
- der Frequenzverteilung oder
- der Frequenz der maximalen Intensität
des statistischen akustischen Rauschens, welches durch die Gasentladung in dem Gas erzeugt wird, bestimmt oder abgeleitet werden. Zu diesem Zweck kann das statistische akustische Rauschen mittels eines akustischen Wandlers, z.B. Mikrofon, erfaßt werden. Der akustische Sensor erfaßt das akustische Rauschen und setzt es in ein drittes Sensorsignal um, aus welchem der Ist-Wert der zwischen den beiden Elektroden bestehenden elektrischen Kapazität bestimmt wird.

Der Ist-Wert der zwischen den beiden Elektroden bestehenden elektrischen Kapazität kann ferner aus
- der integralen Intensität oder
- Intensität mindestens einer bestimmten Wellenlänge oder Wellenlängenbereiches oder
- der mittleren Wellenlänge oder
- der spektralen Wellenlängenverteilung oder
- der Wellenlänge der maximalen Intensität
von elektromagnetischer Strahlung, z.B. Licht, Röntgenstrahlung, Radiostrahlung, die durch die Gasentladung erzeugt und abgestrahlt wird, bestimmt oder abgeleitet werden. Zu diesem Zweck kann ein Strahlungssensor elektromagnetische Strahlung, wie Licht, Röntgenstrahlung oder Radiostrahlung, die durch die Gasentladung erzeugt und abgestrahlt wird, erfassen und in ein viertes Sensorsignal umsetzen und der Ist-Wert der zwischen den beiden Elektroden bestehenden elektrischen Kapazität aus dem vierten Sensorsignal bestimmt werden.

Der Ist-Wert der zwischen den beiden Elektroden bestehenden elektrischen Kapazität kann ferner aus der Phasenverschiebung zwischen der Hochspannung und dem durch diese in Gang gesetzten elektrischen Strom bestimmt oder abgeleitet werden, da diese sich Phasenverschiebung mit der zwischen den beiden Elektroden bestehenden elektrischen Kapazität ändert. Zu diesem Zweck kann ein Phasenvergleicher die Phasenverschiebung zwischen der Hochspannung und dem durch diese in Gang gesetzten elektrischen Strom erfassen und in ein fünftes Sensorsignal umsetzen, wobei der Ist-Wert der zwischen den beiden Elektroden bestehenden elektrischen Kapazität aus dem fünften Sensorsignal bestimmt oder abgeleitet wird.

Zu diesem Zweck wird die Phasenlage des Stromes in Bezug auf die eingespeiste Spannung beobachtet. Es wurde bereits erläutert, daß das Gasentladungsmodul elektrisch als Kondensator zu betrachten ist, welcher in Reihenschaltung mit einem ohmschen Widerstand die Phasenlage des Stromes in Bezug auf die Spannung verändert. Bei den bevorzugt eingesetzten lonisationsmodulen ist die Grundkapazität etwa 5OpF, was bei einem Reihenwiderstand von 100 kOhm und einer Frequenz von ca. 30KHz zu einer Phasenverschiebung von ca. 30° führt.

Wenn das Plasma zündet und sich die Oberfläche durch eine Vielzahl von elektrisch leitenden Filamenten bedeckt, erhöht sich schlagartig und dann weiter zunehmend die Kapazität des durch die Elektroden 3, 4 gebildeten elektrischen Kondensators, was sich auf die Phasenlage auswirkt in dem Sinne, daß die Phasenverschiebung sich verringert und einen minimalen Wert von ca. 10° annimmt.

Erfindungsgemäß wird daher vorgeschlagen, in einem Phasenvergleicher die Phasenlage von Strom und Spannung zu vergleichen und aus den Phasenverschiebungen ein Regelungssignal im vorgenannten Sinne abzuleiten mit dem Ziel, die Phasenlage auf einen bestimmten Wert konstant zu halten. Dieser Wert entspricht einem als optimal definierten Arbeitspunkt.

Der Ist-Wert und der Sollwert der zwischen den beiden Elektroden bestehenden elektrischen Kapazität kann mittels eines Reglers, z.B. PID-Regler, verglichen werden, wobei der Regler aus der Differenz zwischen Ist-Wert und Sollwert oder dem zeitlichen Verhalten dieser Differenz ein zeitlich veränderliches Stellsignal bildet, welches zur Steuerung des Hochspannungsgenerators herangezogen wird. Falls es sich bei dem Gas um Luft handelt, kann der Sollwert vorteilhaft so gewählt sein, daß bei der Gasentladung Ozon und Sauerstoff-Ionen, jedoch kein Stickstoffoxid entsteht.

Ferner wird erfindungsgemäß vorgeschlagen, ein elektrisch und nach dem Prinzip der dielektrisch behinderten Entladung arbeitendes Gasentladungsmodul nach PCT/EP 96/01609, welches entsprechend der Lehre der DE 100 04 326.7 in seinem Arbeitspunkt stabilisiert ist, durch Aufschaltung einer von einem die Gas- bzw. Luftqualität detektierenden Gasqualitätssensor gewonnenen Störgröße situationsadaptiert zu betreiben.

Das Gas kann mittels einer Fördereinrichtung, z.B. Ventilator, so gefördert werden, daß es das Gasentladungsmodul durchströmt. In dem Gasstrom kann ein Gasqualitätssensor so angeordnet sein, daß das Gas den Gasqualitätssensor vor Erreichen des Gasentladungsmoduls passiert, wobei der Gasqualitätssensor ein Gasqualitätssensorsignal abgibt, welches ein Maß für die Belastung des Gases mit Fremdstoffen, Schadstoffen oder Keimen ist und dergestalt zur Beeinflussung des Sollwertes herangezogen wird, daß der Sollwert mit zunehmender bzw. abnehmender Belastung des Gases mit Fremdstoffen, Schadstoffen oder Keimen angehoben bzw. abgesenkt wird.

Gemäß einer Variante der Erfindung befinden sich die Elektroden in einem strömenden Gas, insbesondere Luft, wobei zur Regelung der Ausgangsspannung des Hochspannungsgenerators ein Regler den Spannungs-Ist-Wert mit einem Spannungs-Sollwert vergleicht und, wenn der Spannungs-Ist-Wert unterhalb des Spannungs-Sollwertes liegt, die Hochspannung zeitlich zunehmend gesteuert wird, oder wenn der Spannungs-Ist-Wert oberhalb des Spannungs-Sollwertes liegt, die Hochspannung zeitlich abnehmend gesteuert wird, wobei in dem Gasstrom ein Gasqualitätssensor so angeordnet ist, daß das Gas den Gasqualitätssensor vor Erreichen der Elektroden passiert, wobei der Gasqualitätssensor ein Gasqualitätssensorsignal abgibt, welches ein Maß für die Belastung des Gases mit Fremdstoffen, Schadstoffen oder Keimen ist und dergestalt zur Beeinflussung des Spannungs-Sollwertes herangezogen wird, daß der Spannungs-Sollwert mit zunehmender bzw. abnehmender Belastung des Gases mit Fremdstoffen, Schadstoffen oder Keimen angehoben bzw. abgesenkt wird.

Gemäß einer weiteren Variante befinden sich die Elektroden in einem strömenden Gas, insbesondere Luft, wobei zur Regelung der Ionisisierungsleistung der Gasentladung die Hochspannung über einen dritten Widerstand zwischen den Elektroden angelegt ist und der Ionisierungsleistungs-Ist-Wert aus dem Wert der Hochspannung und der am dritten Widerstand abfallenden Spannung ermittelt wird und ein Regler den Ionisierungsleistungs-Ist-Wert mit einem Ionisierungsleistungs-Sollwert vergleicht und, wenn der Ionisierungsleistungs-Ist-Wert unterhalb des Ionisierungsleistungs-Sollwertes liegt, die Hochspannung zeitlich zunehmend gesteuert wird, oder wenn der Ionisierungsleistungs-Ist-Wert oberhalb des Ionisierungsleistungs-Sollwertes liegt, die Hochspannung zeitlich abnehmend gesteuert wird, wobei in dem Gasstrom ein Gasqualitätssensor so angeordnet ist, daß das Gas den Gasqualitätssensor vor Erreichen der Elektroden passiert, wobei der Gasqualitätssensor ein Gasqualitätssensorsignal abgibt, welches ein Maß für die Belastung des Gases mit Fremdstoffen, Schadstoffen oder Keimen ist und dergestalt zur Beeinflussung des Spannungs-Sollwertes herangezogen wird, daß der Ionisierungsleistungs-Sollwert mit zunehmender bzw. abnehmender Belastung des Gases mit Fremdstoffen, Schadstoffen oder Keimen angehoben bzw. abgesenkt wird.

Der Gasqualitätssensor kann insbesondere ein Sensor für Gerüche oder Verkeimung oder für gasförmige oder partikelförmige Fremd- oder Schadstoffe sein. In einer bevorzugten Ausführungsform ist das Gas Luft und der Gasqualitätssensor ein Sensor für oxidierbare in der Luft enthaltene gasförmige oder partikelförmige Fremd- oder Schadstoffe. Der Gasqualitätssensor kann eine geregelte Heizung aufweisen, so daß der Gasqualitätssensor immer eine konstante Temperatur aufweist.

Das Gasqualitätssensorsignal kann erfindungsgemäß gleichzeitig genutzt werden, um ein luftqualitätsbezogenes Schaltsignal zu erzeugen, welches dann, wenn eine erheblich erhöhte Schadstoffkonzentration der Luft detektiert wird, durch Eingreifen in die Lenkung der Luftströme das Einbringen der Schadstoffe in Gebäude, Räume oder Fahrzeugkabinen zu verhindern. Es kann auch die Förderleistung der lufttechnischen Anlage als Funktion der Luftqualität beeinflußt werden. Das Sensorsignal kann weiter genutzt werden, um die Funktion und Leistungsfähigkeit eines im Lufteinlaß angeordneten Luftfilters zu bewerten.

Die Führung des Gasstromes kann vorteilhaft in Abhängigkeit von der vom Gasqualitätssensor gemessenen Konzentration von Fremdstoffen, Schadstoffen oder Keimen beeinflußt werden. Z.B. kann das Gasqualitätssensorsignal kann dazu herangezogen werden, das Gas in einen Zulauf-Ablauf-Betrieb zu versetzen, in welchem es dem Gasqualitätssensor von außen z.B. durch eine Einlaßöffnung zugeführt wird und nach Passieren der Elektroden z.B. durch einen Auslaß wieder nach außen abgeführt wird, wenn die Konzentration von Fremdstoffen, Schadstoffen oder Keimen in dem Gas einen bestimmten Grenzwert übersteigt bzw. unterschreitet, oder das Gas in eine Umlauf-Zirkulation zu versetzen, in welcher das Gas nach Passieren der Elektroden in einem geschlossenen Kreislauf erneut an den Gasqualitätssensor geführt wird, wenn die Konzentration von Fremdstoffen, Schadstoffen oder Keimen in dem Gas einen bestimmten Grenzwert unterschreitet bzw. übersteigt.

Dieses Prinzip läßt sich z.B. vorteilhaft auf die Belüftung von Kraftfahrzeugen anwenden: bei schlechter Außenluft kann auf Umluft umgeschaltet werden, wobei das Gasentladungsmodul die nun in einer Umlauf-Zirkulation strömende Luft umso intensiver aufbereitet, je größer die Konzentration von Fremdstoffen, Schadstoffen oder Keimen in der Luft ist.

Wenn die Konzentration von Fremdstoffen, Schadstoffen oder Keimen in dem Gas, nachdem das Gas in die Umlauf-Zirkluation bzw. in den Zulauf-Ablauf-Betrieb versetzt wurde, weiterhin ansteigt, kann hierbei das Gas wieder zurück in den Zulauf-Ablauf-Betrieb bzw. zurück in die Umlauf-Zirkulation versetzt werden. Auf diese Weise kann z.B. bei der Belüftung eines Kfz verhindert werden, daß bei Umluftbetrieb die Luftqualität aufgrund einer hinreichend intensiven Kfz-internen Schadstoffquelle, deren zu Schadstoffausstoß groß ist, um durch die Funktion des Gasentladungsmoduls ausgeglichen zu werden, immer weiter absinkt.

In einer weiteren Ausgestaltung der Erfindung wird das Gas bei Zulauf-Ablauf-Betrieb nach einer bestimmten Zeit in die Umlauf-Zirkulation versetzt, wobei der Gasqualitätssensor unmittelbar vor dem Übergang in die Umlauf-Zirkulation einen ersten Meßwert und unmittelbar nach dem Übergang einen zweiten Meßwert für die Konzentration von Fremdstoffen, Schadstoffen oder Keimen liefert und das Gas nur dann wieder in den Zulauf-Ablauf-Betrieb zurückversetzt wird, wenn der erste Meßwert niedriger ist als der zweite Meßwert. Analog hierzu wird das Gas bei Umlauf-Zirkulation nach der bestimmten Zeit in den Zulauf-Ablauf-Betrieb versetzt, wobei der Gasqualitätssensor unmittelbar vor dem Übergang in den Zulauf-Ablauf-Betrieb einen dritten Meßwert und unmittelbar nach dem Übergang einen vierten Meßwert für die Konzentration von Fremdstoffen, Schadstoffen oder Keimen liefert und das Gas nur dann wieder in die Umlauf-Zirkulation zurückversetzt wird, wenn der dritte Meßwert niedriger ist als der vierte Meßwert.

Dies bedeutet, daß das Gas erfindungsgemäß entweder in den Zulauf-Ablauf-Betrieb oder in die Umlauf-Zirkulation versetzt wird, je nachdem, welche dieser beiden Betriebsarten mit weniger Belastung des Gases durch Fremdstoffe, Schadstoffe oder Keime verbunden ist, und daß die Entscheidung über die zu wählende Betriebsart in bestimmten Zeitabständen wiederholt wird.

Um immer die niedrigste mögliche Konzentration an Fremdstoffen, Schadstoffen oder Keimen zu gewährleiste, kann der Grenzwert, bei welchem zeitunabhängig ein Übergang vom Zulauf-Ablauf-Betrieb zur Umlauf-Zirkulation bzw. umgekehrt erfolgt, ein gleitender Grenzwert sein. Z.B. kann zu diesem Zweck der erste Meßwert mit dem zweiten Meßwert verglichen werden und als Grenzwert der niedrigere dieser beiden Meßwerte übernommen werden, bzw. der dritte Meßwert mit dem vierten Meßwert verglichen werden und als Grenzwert der niedrigere dieser beiden Meßwerte übernommen werden, so daß der Grenzwert den in bestimmten Zeitabständen durch einen neuen Grenzwert ersetzt wird. Bei der Belüftung eines Kfz z.B. kann auf diese Weise der Grenzwert in Abhängigkeit von der tatsächlich in und außerhalb des Kfz vorliegenden Luftbelastung nachgeführt werden.

Das Gasqualitätssensorsignal kann ferner dazu herangezogen werden, mit zunehmender Konzentration von Fremdstoffen, Schadstoffen oder Keimen in dem Gas die Strömungsgeschwindigkeit des Gases zu vergrößern und umgekehrt. Auf diese Weise kann z.B. bei großer Luftbelastung die Fördermenge pro Zeiteinheit erhöht werden, um die Schadstoffe, Keime usw. möglichst rasch abzuführen.

Die Störeinflüsse von Luftfeuchte, Strömungsgeschwindigkeit und Gastemperatur auf das Gasqualitätssensorsignal können rechnerisch kompensiert werden, so daß das Gasqualitätssensorsignal immer ein eindeutiges und reproduzierbares Maß für die tatsächlich vorliegende Konzentration von Fremdstoffen, Schadstoffen oder Keimen in dem Gas ist. In diesem Fall kann die Konzentration von Fremdstoffen, Schadstoffen oder Keimen nach der Kompensation der Störeinflüsse von Luftfeuchte, Strömungsgeschwindigkeit und Gastemperatur vorteilhaft auf einem Display angezeigt werden, so daß ungeachtet der Störeinflüsse von Luftfeuchte, Strömungsgeschwindigkeit und Gastemperatur die tatsächlich vorliegende Konzentration von Fremdstoffen, Schadstoffen oder Keimen jederzeit ablesbar ist.

Gemäß einer weiteren Variante des Verfahrens ist im Gasstrom ein Gasfilter, z.B. Luftfilter, angeordnet, dessen Fähigkeit, ein dem Gasstrom zugesetztes Prüfgas zu adsorbieren, mit steigender Erschöpfung des Gasfilters abnimmt, wobei der Gasstrom nach Durchlaufen des Gasfilters einen Gasqualitätssensor passiert, welcher das Prüfgas zu detektieren imstande ist und ein Gasqualitätssensorsignal abgibt, das ein Maß für die Konzentration des Prüfgases ist, das Prüfgas dem Gasstrom vor Erreichen des Gasfilters für eine bestimmte definierte Zeitspanne in einer konstanten Konzentration zugesetzt wird, und der Erschöpfungsgrad des Gasfilters unter Ausnutzung des Umstandes, daß die Steilheit des zeitlichen Anstieges des Gasqualitätssensorsignals nach Beginn und des zeitlichen Gefälles des Gasqualitätssensorsignals nach Ende des Zusetzens des Prüfgases mit dem Erschöpfungsgrad des Gasfilters abnehmen, aus der Steilheit dieser Steigung bzw. dieses Gefälles bestimmt wird.

### Kurzbeschreibung der Zeichnung, in welcher zeigen:

- Fig. 1: ein Diagramm, welches schematisch den Zusammenhang zwischen der an ein Gasentladungsmodul angelegten Wechselspannung und dem durch diese in Gang gesetzten Strom veranschaulicht,
- Fig. 2: die Abhängigkeit des durch eine sinusförmige Hochspannung in Gang gesetzten Stromes von der Zeit und die Entstehung des elektrischen Rauschens bei einer ersten Größe der Wechselspannung,
- Fig. 3: die Abhängigkeit des durch eine sinusförmige Hochspannung in Gang gesetzten Stromes von der Zeit und die Entstehung des elektrischen Rauschens bei einer zweiten Größe der Wechselspannung,
- Fig. 4: eine Ausführungsform einer erfindungsgemäßen Vorrichtung, in welcher der Ist-Wert der zwischen den Elektroden bestehenden Kapazität aus dem durch die Hochspannung in Gang gesetzte Strom bestimmt wird,
- Fig. 5: eine Ausführungsform einer erfindungsgemäßen Vorrichtung, in welcher der Ist-Wert der zwischen den Elektroden bestehenden Kapazität aus dem elektrischen Rauschen des durch die Hochspannung in Gang gesetzten Stromes bestimmt wird,
- Fig. 6: eine Ausführungsform einer erfindungsgemäßen Vorrichtung, in welcher der Ist-Wert der zwischen den Elektroden bestehenden Kapazität aus dem durch die Gasentladung verursachten akustischen Rauschen bestimmt wird,
- Fig. 7: eine Ausführungsform einer erfindungsgemäßen Vorrichtung, in welcher der Ist-Wert der zwischen den Elektroden bestehenden Kapazität aus dem der durch die Gasentladung verursachten Lichtemission bestimmt wird,
- Fig. 8: eine Ausführungsform einer erfindungsgemäßen Vorrichtung, bei welcher der Sollwert der zwischen den Elektroden bestehenden Kapazität mittels einer Störgrößenaufschaltung durch die Belastung des Gases mit Schadstoffen oder Keimen beeinflußt wird,
- Fig. 9: eine Ausführungsform der Erfindung, welche zur Steuerung der Luftführung in der Klimaanlage eines Kfz eingesetzt ist, und
- Fig. 10: ein Diagramm des zeitlichen Verlaufes der Konzentrationen eines Prüfgases vor und nach Durchlaufen eines Filters.

Zur weiteren Erläuterung des der Erfindung zu Grunde liegenden Prinzips wird zunächst nochmals auf die Figuren 1-3 Bezug genommen.

Fig 1 zeigt ein Diagramm, welches schematisch den Zusammenhang zwischen der an ein Gasentladungsmodul angelegten Wechselspannung und dem durch diese in Gang gesetzten Strom veranschaulicht. Der Kurvenabschnitt a stellt denjenigen Bereich dar, in welchem die Größe der Wechselspannung nicht ausreicht, ein Plasma zu erzeugen und damit eine Gasentladung in Gang zu setzen. Die Elektroden des Gasentladungsmoduls bilden einen Kondensator mit einer festen Kapazität, so daß die Wechselspannungsquelle nur durch den kapazitiven Widerstand dieser Kapazität belastet ist. Daher verläuft der Kurvenabschnitt a von Fig. 1 streng linear.

Wenn die Zündspannung erreicht ist, bildet sich ein Plasma aus. Die Bildung des Plasmas bewirkt eine Vergrößerung der effektiven als Elektrode wirkenden Fläche und daher eine Vergrößerung der zwischen den Elektroden bestehenden elektrischen Kapazität, so daß der Strom überproportional zur Spannung zunimmt (Kurvenabschnitte b, c von Fig.1).

Fig. 2 zeigt die Abhängigkeit des durch eine sinusförmige Hochspannung in Gang gesetzten Stromes von der Phase und die Entstehung des elektrischen Rauschens bei einer ersten Größe der Wechselspannung. Solange kein Plasma zündet, weist der Strom eine Sinusform 20 auf. Die Spannung ist in Fig. 2 so gewählt, daß in einem Bereich um den Scheitel (90°) der Sinuswelle, ca. von einer Phase von 70° bis zu einer Phase von 130°, eine Zündung des Plasmas erfolgt. Das Zünden des Plasmas macht sich durch eine schnell fluktuierende Zunahme des Stromes bemerkbar, die sich der Sinuswelle 20 des Stromes als Rauschen überlagert. Diese die Sinuswelle 20 überlagernde Fluktuation ist in Fig. 2 durch ein Band 21 veranschaulicht, welches auf dem Scheitelbereich der Sinuswelle 20 aufliegt und nur für die Halbwelle mit der Phase 0°-180° in Fig. 2 eingezeichnet ist. Die Breite des Bandes 21 entspricht der maximal auftretenden Größe der Fluktuation.

Fig. 3 zeigt die Abhängigkeit des durch eine sinusförmige Hochspannung in Gang gesetzten Stromes von der Phase und die Entstehung des elektrischen Rauschens bei einer zweiten Größe der Wechselspannung. Die Spannung ist in Fig. 3 so gewählt, daß in einem Bereich um den Scheitel (90°) der Sinuswelle, ca. von einer Phase von 88° bis zu einer Phase von 110°, eine Zündung des Plasmas erfolgt. Die dadurch ausgelöste, die Sinuswelle 22 überlagernde Fluktuation des Stromes ist in Fig. 3 durch ein schwarzes Band 23 veranschaulicht und nur für die Halbwelle mit der Phase 0°-180° eingezeichnet.

Fig. 4 zeigt eine Ausführungsform einer erfindungsgemäßen Vorrichtung, in welcher der Ist-Wert der zwischen den Elektroden bestehenden Kapazität aus dem durch die Hochspannung in Gang gesetzte Strom bestimmt wird. Ein Hochspannungsgenerator 1 gibt eine Wechselspannung an die Elektroden 3, 4 eines Gasentladungsmoduls 2 ab, so daß die Wechselspannung zwischen den Elektroden 3, 4 anliegt. Der Hochspannungsgenerator 1 weist einen Steuereingang 1a auf und ist durch ein Signal, welches an den Steuereingang 1a angelegt wird, steuerbar. Gas 5 kann durch einen Einlaß 14 in das Gasentladungsmodul 2 einströmen und dieses durch einen Auslaß 15 verlassen; hierbei kann es z.B. mit Hilfe eines Ventilators (nicht gezeigt) gefördert werden. Das Gasentladungsmodul 2 hat die Aufgabe, das Gas 5 aufzubereiten, z.B. von Keimen und Gerüchen zu befreien.

Zwischen den Elektroden 3, 4 besteht eine elektrische Kapazität, welche einen festen Wert besitzt, solange die Wechselspannung zu klein ist, um in dem Gasentladungsmodul 2 ein Plasma zu zünden. Aufgrund des kapazitiven Widerstandes dieser Kapazität setzt die Wechselspannung einen elektrischen Strom ist Gang, welcher im folgenden Ruhestrom genannt wird.

Die zwischen den Elektroden bestehende elektrische Kapazität steigt von dem festen Wert aus an und ist schnellen Fluktuationen unterworfen, sobald die Wechselspannung groß genug ist, um in dem Gasentladungsmodul 2 ein Plasma zu zünden. Daher steigt in diesem Fall auch der Stromfluß über den Ruhestrom hinaus an und kann zur Bestimmung des Ist-Wertes der zwischen den Elektroden bestehenden elektrischen Kapazität herangezogen werden. Zu diesem Zweck sind der Stromfluß in Fig. 4 über einen ersten Widerstand R1 geführt und die am ersten Widerstand abfallende Spannung mittels zweier Leitungen 10a, 10b abgegriffen und an den Ist-Wert-Eingang 6a eines Reglers 6 angelegt.

Der erste Widerstand R1 fungiert somit als Sensor für den durch die Hochspannung in Gang gesetzten elektrischen Strom und setzt diesen in ein erstes Sensorsignal um, welches dem Ist-Wert der zwischen den beiden Elektroden bestehenden elektrischen Kapazität entspricht.

Der Regler 6 vergleicht die am Ist-Wert-Eingang 6a anliegende Spannung mit einem Sollwert, der z.B. über einen weiteren Eingang (nicht gezeigt) in den Regler eingegeben wird oder intern im Regler gespeichert ist oder berechnet wird, und gibt erfindungsgemäß über einen Steuerausgang 6b ein solches Steuersignal an den Steuereingang 1a ab, daß wenn der Ist-Wert der zwischen den beiden Elektroden 3, 4 bestehenden Kapazität unterhalb eines bestimmten Sollwertes liegt, der Effektivwert der Hochspannung des Hochspannungsgenerators 1 zeitlich zunehmend gesteuert wird, oder wenn der Ist-Wert der zwischen den beiden Elektroden 3, 4 bestehenden effektiven elektrischen Kapazität oberhalb eines bestimmten Sollwertes liegt, der Effektivwert der Hochspannung des Hochspannungsgenerators 1 zeitlich abnehmend gesteuert wird.

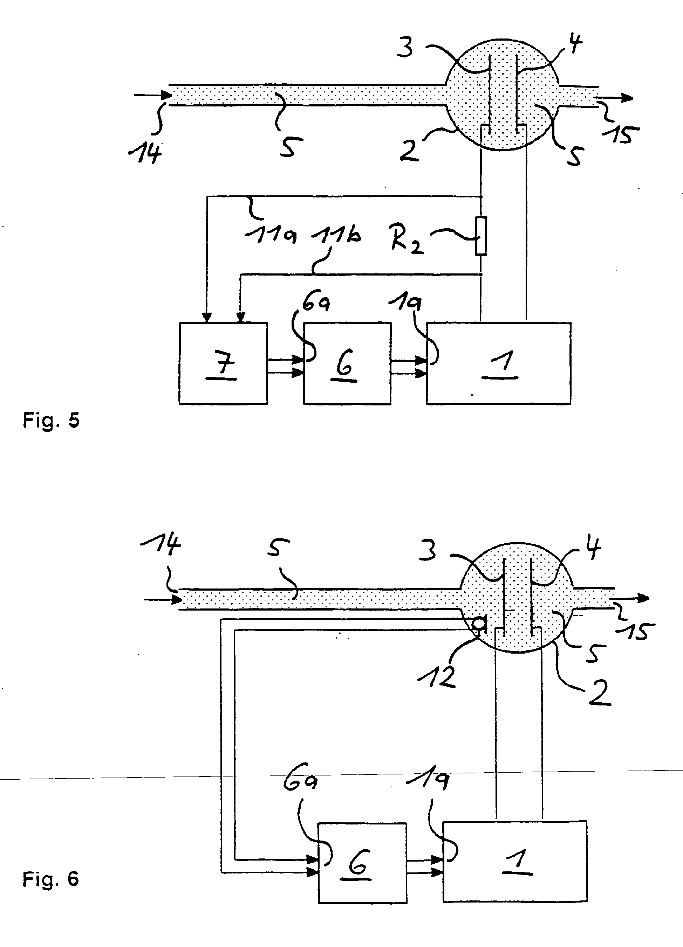

Fig. 5 veranschaulicht eine Ausführungsform einer erfindungsgemäßen Vorrichtung, in welcher der Ist-Wert der zwischen den Elektroden 3, 4 bestehenden Kapazität aus dem elektrischen Rauschen des durch die Hochspannung in Gang gesetzten Stromes bestimmt wird. Die Vorrichtung von Fig. 5 unterscheidet sich von derjenigen von Fig. 4 dadurch, daß in Fig. 5 zusätzlich ein frequenzselektives Filter 7 vorhanden ist und der Stromfluß nicht über den ersten Widerstand R1, sondern über einen zweiten Widerstand R2 geführt wird. Die dort abfallende Spannung wird mittels zweier Leitungen 11a, 11b abgegriffen und über das frequenzselektive Filter 7 an den Ist-Wert-Eingang 6a des Reglers 6 geführt, der den Hochspannungsgenerator 1 steuert wie unter Bezug auf Fig. 4 erläutert. Das frequenzselektive Filter 7 kann z.B. ein Bandpaß oder ein Hochpaß sein.

Eine der erfindungsgemäßen Möglichikeiten zu einer Regelung ist es demnach, das durch Zünden und das kurzzeitige Brennen der Filamente verursachte elektrische "Rauschen" als Regelgröße auszunutzen. Dies, weil sich bei geringer Spannung entweder keine oder nur geringe Rauschsignale bilden können, was im Falle einer auf dieser Rauschspannung basierenden Regelung die Folge hat, daß die Wechselspannung erhöht wird. Technisch erfolgt dies, indem durch ein auf das hochfrequente Rauschspektrum abgestimmtes Filter 7 oder durch ein Hochpaß-Frequenzfilter die hochfrequente Rauschspannung von der Frequenz der Wechselspannung abgetrennt wird, welche typisch bei 15- 35KHz betrieben wird. Die so abtrennte Rauschspannung kann durch Demodulation und durch weitere Glättung zu einer Regelspannung aufbereitet werden. Diese Regelspannung wird dem Hochspannungsgenerator 1 in einem Sinne zugeführt, daß bei hohem Rauschanteil (= hohe Regelspannung) die Hochspannung verringert wird und bei geringem Rauschanteil die Hochspannung erhöht wird. Ergebnismäßig wird unter Einwirkung einer Rauschanteil-/ Spannungsregelung der Betrieb des Gasentladungsmoduls 2 in einem vorgegebenen, stets konstanten Bereich betrieben werden mit der Folge einer stets konstanten Produktionsmenge von Sauerstoffionen und Ozon.

In der Vorrichtung von Fig. 5 wird also der Effekt ausgenutzt, daß die zwischen den Elektroden 3, 4 bestehende Kapazität schnellen Fluktuationen unterworfen ist, sobald die Wechselspannung groß genug ist, um im Gasentladungsmodul 2 ein Plasma zu zünden, und daß diese Fluktuationen mit zunehmender Kapazität weiter ansteigen. Daher ist in diesem Fall auch der durch die Wechselspannung in Gang gesetzte elektrische Strom schnellen Fluktuationen unterworfen, was sich als ein dem Ruhestrom überlagertes elektrisches Rauschen bemerkbar macht, welches mit zunehmender Kapazität weiter ansteigt und daher als Maß für den Ist-Wert der Kapazität verwendet werden kann. In einer Variante dieses Prinzips (nicht gezeigt) wird der zweite Widerstand R2, der eine aperiodische Auskopplung der Rauschspannung ermöglicht, durch eine elektrische Spule (Induktivität) ersetzt. Vorteilhaft ist bei dieser Variante, daß das gewonnene Signal eine höhere Amplitude gegenüber der Speisefrequenz aufweist. In einer Weiterführung dieser Variante ist die Spule ersetzt durch einen aus einer Spule und einem Kondensator bestehenden Schwingkreis, welcher auf die Mittenfrequenz des Rauschsignals abgestimmt ist.

Fig. 6 veranschaulicht eine Ausführungsform einer erfindungsgemäßen Vorrichtung, in welcher der Ist-Wert der zwischen den Elektroden 3, 4 bestehenden Kapazität aus dem akustischen Rauschen bestimmt wird, welches die Gasentladung in dem Gas 5 verursacht. Das Gasentladungsmodul 2 weist ein Mikrofon 12 auf, welches Schallwellen im Gas 5 erfaßt und als Mikrofonsignal ausgibt. Das Mikrofonsignal wird - nötigenfalls nach Durchlaufen eines Verstärkers (nicht gezeigt) an den Ist-Wert-Eingang 6a des Reglers 6 geführt, der den Hochspannungsgenerator 1 steuert wie unter Bezug auf Fig. 4 erläutert.

In der Vorrichtung von Fig. 6 wird der Effekt ausgenutzt, daß die schnellen Fluktuationen der zwischen den Elektroden 3, 4 bestehenden Kapazität Schallwellen oder Ultraschallwellen im Gas 5 auslösen, und daß diese Fluktuationen mit zunehmender Kapazität weiter ansteigen und mit Eigenschaften der Schallwellen wie Intensität, Frequenzverteilung, Änderungsrate usw. korrelieren. Aus dem Mirkofonsignal kann daher der Ist-Wert der Kapazität bestimmt oder abgeleitet werden kann.

In einer Variante der Vorrichtung von Fig. 6 (nicht gezeigt) durchläuft das Mikrofonsignal vor Erreichen des Ist-Wert-Einganges 6a ein frequenzselektives Filter, das z.B. ein Bandpaß oder ein Hochpaß sein kann.

In weiteren Varianten der Erfindung können das elektrische oder das akustische Rauschen durch Spektralanalyse weiter zerlegt werden. Die Verteilung im Spektrum gibt Aufschlüsse über den Arbeitsbereich des Gasentladungsmoduls (Qualitative Auswertung des Spektrums).

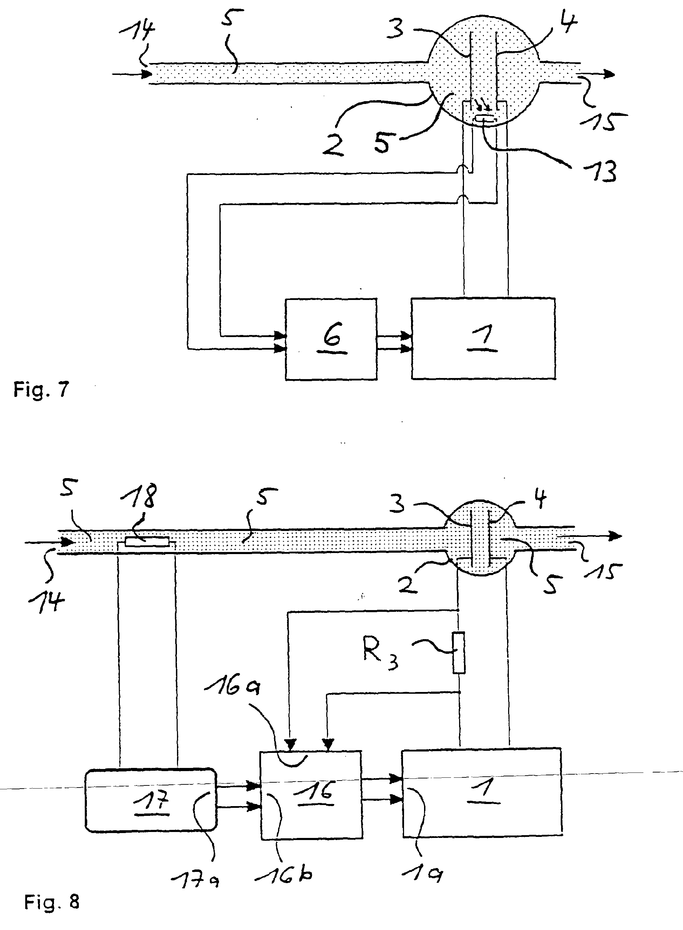

Fig. 7 veranschaulicht eine Ausführungsform einer erfindungsgemäßen Vorrichtung, in welcher der Ist-Wert der zwischen den Elektroden bestehenden Kapazität aus dem der durch die Gasentladung verursachten Lichtemission bestimmt wird. Am oder im Gasentladungsmodul 2 ist ein Fotodetektor 13 angeordnet, welcher Licht, das bei der Gasentladung abgestrahlt wird, erfaßt und als Fotodetektorsignal ausgibt. Das Fotodetektorsignal wird - nötigenfalls nach Durchlaufen eines Verstärkers (nicht gezeigt) an den Ist-Wert-Eingang 6a des Reglers 6 geführt, der den Hochspannungsgenerator 1 steuert wie unter Bezug auf Fig. 4 erläutert.

In der Vorrichtung von Fig. 7 wird der Effekt ausgenutzt, daß bei der Gasentladung Licht emittiert wird, welches aufgrund der schnellen Fluktuationen der zwischen den Elektroden 3, 4 bestehenden Kapazität ebenfalls fluktuiert, und daß diese Fluktuationen mit zunehmender Kapazität weiter ansteigen und mit Eigenschaften des emittierten Lichts wie Intensität, Frequenzverteilung, Änderungsrate usw. korrelieren. Aus dem Fotodetektorsignal kann daher der Ist-Wert der Kapazität bestimmt oder abgeleitet werden.

Die hier genannten Methoden sind beispielhaft und können selbstverständlich weiter variiert werden. Gemeinsam ist jedoch sämtlichen der unter Bezug auf Fig. 3 bis Fig. 7 erläuterten Methoden, daß elektrische Parameter, welche sich beim Betrieb von Gasentladungsmodulen bei einer Veränderung des Betriebspunktes ändern, derart einer Auswertung zugeführt werden mit dem Ziel, einen geschlossenen Regelkreis zu bilden, der den Betriebspunkt des Gasentladungsmoduls dann konstant hält, unabhängig von sonstigen Einflüssen, wie Luftfeuchte, Strömungsgeschwindigkeit der Luft, Belastung der Luft mit Luftinhaltsstoffen als auch von fertigungsbedingten Toleranzen der elektrischen Werte des Gasentladungsmoduls.

Fig. 8 zeigt eine Ausführungsform der Erfindung, bei welcher der Sollwert der zwischen den Elektroden 3, 4 eines Gasentladungsmoduls 2 bestehenden Kapazität durch die Belastung des Gases 5 mit Schadstoffen oder Keimen beeinflußt wird. Diese Beeinflussung erfolgt mittels einer Störgrößenaufschaltung. Der Hochspannungsgenerator 1 gibt eine Hochspannung ab, welche über einen dritten Widerstand R3 an die Elektroden 3, 4 des Gasentladungsmoduls 2 angelegt ist. Der durch die Hochspannung in Gang gesetzte Stromfluß führt zu einem Spannungsabfall am Widerstand R3, welcher abgegriffen und an den Ist-Wert-Eingang 16a eines Reglers 16 angelegt wird, der darüber hinaus einen Sollwert-Eingang 16b aufweist. Analog zu der unter Bezug auf Fig. 4 erläuterten Vorrichtung wird die zwischen den beiden Elektroden bestehende elektrische Kapazität über den durch die Hochspannung in Gang gesetzten Stromfluß erfaßt und mittels eines Reglers, welcher Ist-Wert und Sollwert vergleicht, geregelt. Der Ist-Wert wird, wie in Fig. 4, aus dem Stromfluß ermittelt.

Im Gegensatz zu der Vorrichtung von Fig. 4 weist jedoch die Vorrichtung von Fig. 8 einen Regler 16 auf, welcher einen Ist-Wert-Eingang 16a und einen Sollwert-Eingang 16b besitzt. Der Sollwert wird, z.B. in Form einer elektrischen Spannung, vom Ausgang 17a einer Steuereinheit 17, die insbesondere ein Mikroprozessor sein kann, abgegeben und an den Sollwert-Eingang 16b des Reglers 16 geführt.

An die Steuereinheit 17 ist ein Gasqualitätssensor 18 angeschlossen, der im Strom des Gases 5 so angeordnet ist, daß das Gas 5 den Gasqualitätssensor 18 passiert, bevor es das Gasentladungsmodul 2 erreicht.

Der Gasqualitätssensor 18 gibt ein Gasqualitätssensorsignal ab, welches ein Maß für die Konzentration von Fremdstoffen, Schadstoffen oder Keimen in dem Gas 5 ist und der Steuereinheit 17 zugeführt wird. Die Steuereinheit 17 steuert den Sollwert an ihrem Ausgang 17b erfindungsgemäß so, daß der Sollwert der Kapazität und damit der Effektivwert der Hochspannung angehoben wird, wenn die Konzentration von Fremdstoffen, Schadstoffen oder Keimen in dem Gas 5 steigt, und daß im umgekehrten Fall der Sollwert fällt. Die Konzentration von Fremdstoffen, Schadstoffen oder Keimen wird somit zur Bildung einer Störgröße für den Sollwert herangezogen. In einer bevorzugten Ausführungsform gehört dabei zu jeder Konzentration von Fremdstoffen, Schadstoffen oder Keimen in dem Gas 5 in umkehrbar-eindeutiger Zuordnung ein bestimmter Sollwert.

Auf diese Weise wird vorteilhafterweise erreicht, daß der Sollwert der zwischen den Elektroden 3, 4 bestehende Kapazität bei starker Belastung des Gases 5 Fremdstoffen, Schadstoffen oder Keimen angehoben wird, so daß eine intensivere Gasentladung und damit eine intensivere Aufbereitung des Gases 5 die Folge ist, und umgekehrt.

In weiteren Varianten dieser Ausführungsform der Erfindung wird der Ist-Wert nicht aus dem Stromfluß abgeleitet, sondern aus dem elektrischen Rauschen des durch die Hochspannung in Gang gesetzten Stromes oder aus dem durch die Gasentladung ausgelösten akustischen Rauschen oder aus elektromagnetischer Strahlung, welche bei der Gasentladung emittiert wird.

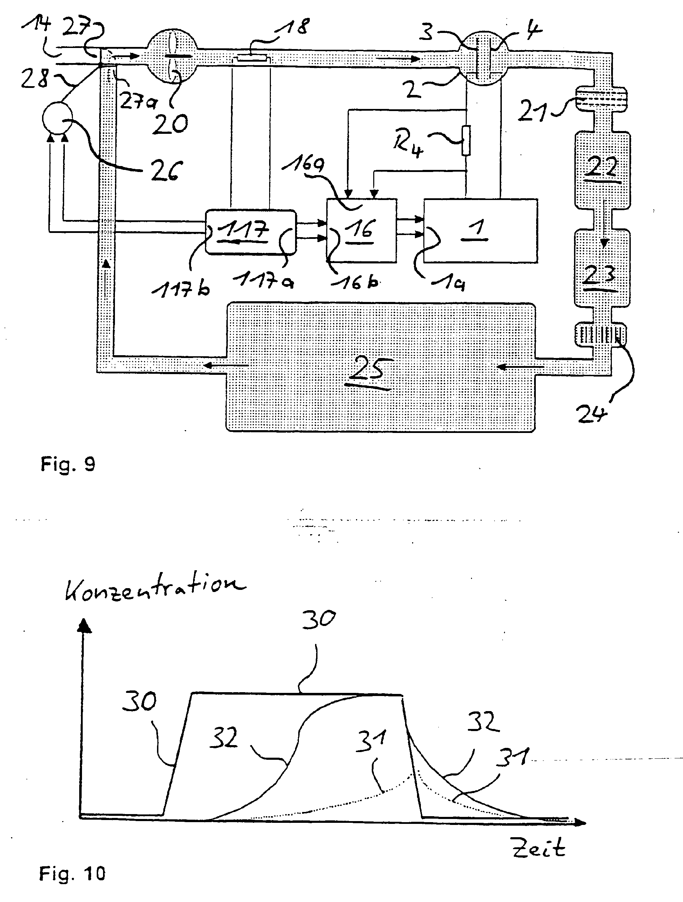

Fig. 9 zeigt eine Ausführungsform der Erfindung, welche zur Steuerung der Luftführung in der Klimaanlage eines Kfz eingesetzt ist. Das Gas ist Luft, welche sich in dem in Fig. 9 veranschaulichten Betriebszustand in Umlauf-Zirkulation befindet. Ein Ventilator 20 versetzt die Luft in Strömung, wobei die Pfeile in Fig. 9 die Richtung der Luftströmung angeben. Die vom Ventilator 20 geförderte Luft passiert zunächst den Gasqualitätssensor 18 und danach das Gasentladungsmodul 2 mit den Elektroden 3, 4. Von dort wird die Luft nacheinander durch ein Luftfilter 21, durch einen Heizer 22 und durch einen Kühler 23, durch einen Katalysator 24 in die Kabine 25 des Kfz geführt. Von dort wird die Luft zurückgeführt an den Ventilator 20, wo sie einen neuen Zyklus der Umlauf-Zirkulation beginnt.

Mit Hilfe eines Antriebs 26, welcher über einen Stellmechanismus 28 auf die Luftklappe 27 einwirkt, kann die Luftklappe 27 in eine Stellung 27a verschwenkt und damit von der Umlauf-Zirkulation in einen Zulauf-Ablauf-Betrieb übergegangen werden, bei welchem der Kreislauf der Luft durch die Luftklappe 27 unterbrochen ist und Außenluft durch den Einlaß 14 einströmt; ein Auslaß ist in Fig. 9 nicht gezeigt, da in der Praxis vorhandene Öffnungen und Undichtigkeiten der Kabine 25 als Auslaß fungieren können.

Der Hochspannungsgenerator 1 gibt eine Hochspannung ab, welche über einen vierten Widerstand R4 an die Elektroden 3, 4 des Gasentladungsmoduls 2 angelegt ist. Der durch die Hochspannung in Gang gesetzte Stromfluß führt zu einem Spannungsabfall am Widerstand R4, welcher abgegriffen und an den Ist-Wert-Eingang 16a des Reglers 16 angelegt wird, der darüber hinaus einen Sollwert-Eingang 16b aufweist. Analog zu der unter Bezug auf Fig. 4 erläuterten Vorrichtung wird die zwischen den beiden Elektroden bestehende elektrische Kapazität über den durch die Hochspannung in Gang gesetzten Stromfluß erfaßt und mittels eines Reglers, welcher Ist-Wert und Sollwert vergleicht, geregelt. Der Ist-Wert wird, wie in Fig. 4, aus dem Stromfluß ermittelt.

Der Regler 16 weist einen Ist-Wert-Eingang 16a und einen Sollwert-Eingang 16b auf, wobei der Sollwert z.B. in Form einer elektrischen Spannung vom Ausgang 117a einer Steuereinheit 117, die insbesondere ein Mikroprozessor sein kann, abgegeben und an den Sollwert-Eingang 16b des Reglers 16 geführt wird. Die Steuereinheit 117 weist einen ersten Ausgang 117a und einen zweiten Ausgang 117b auf.

Der Gasqualitätssensor 18 gibt ein Gasqualitätssensorsignal ab, welches ein Maß für die Konzentration von Fremdstoffen, Schadstoffen oder Keimen in der Luft ist und der Steuereinheit 117 zugeführt wird. Die Steuereinheit 117 steuert den Sollwert an ihrem Ausgang 117b erfindungsgemäß so, daß der Sollwert der Kapazität und damit der Effektivwert der Hochspannung angehoben wird, wenn die Konzentration von Fremdstoffen, Schadstoffen oder Keimen in der Luft steigt, und daß im umgekehrten Fall der Sollwert fällt. Die Konzentration von Fremdstoffen, Schadstoffen oder Keimen wird somit zur Bildung einer Störgröße für den Sollwert herangezogen.

Das Gasqualitätssensorsignal wird in der in Fig. 9 veranschaulichten Ausführungsform der Erfindung des Weiteren dazu herangezogen, die Luft in den Zulauf-Ablauf-Betrieb zu versetzen, wenn die Konzentration von Fremdstoffen, Schadstoffen oder Keimen in der Luft einen bestimmten Grenzwert übersteigt bzw. unterschreitet, oder die Luft in die Umlauf-Zirkulation zu versetzen, wenn die Konzentration von Fremdstoffen, Schadstoffen oder Keimen in dem Gas einen bestimmten Grenzwert unterschreitet bzw. übersteigt. Zu diesem Zweck weist die Steuereinheit 117 einen zweiten Ausgang 117b auf, über welchen der Antrieb 26 so gesteuert wird, daß dieser durch entsprechendes Verschwenken der Luftklappe 27 die Luft in die Umlauf-Zirkulation versetzt, wenn der Grenzwert überschritten ist, oder in den Zulauf-Ablauf-Betrieb versetzt, wenn der Grenzwert nicht überschritten ist bzw. umgekehrt. Somit dient das Gasqualitätssensorsignal erfindungsgemäß sowohl zur Beeinflussung des Sollwertes als auch zur Steuerung der Luftführung.

Gemäß einer weiteren Ausführungsform der Erfindung wird das Gasentladungsmodul vom Hochspannungsgenerator mit Hochspannung vorzugsweise einer Größenordnung von 4-6 kV und einer Frequenz von 20-40 kHz versorgt. Der das Gasentladungsmodul durchfließende Strom fließt durch eine elektrische Spule oder durch einen elektrischen Widerstand. In diesem so gebildeten Spannungsteiler wird ein für die Ionisationsstärke typisches Signal abgegriffen und über einen Regler geleitet, so daß eine konstante lonisationsleistung entsteht nach folgender Logik:
- Weniger Ionisationsleistung als vorgegeben =: Hochspannung anheben
- Mehr Ionisationsleistung als vorgegeben =: Hochspannung absenken

Vom Ergebnis her ist durch diese Methode die Entladungstätigkeit des Ionisatonsmoduls weitgehend konstant, und dies unabhängig von klimatischen Einflüssen oder von der Exemplartoleranz der Ionisationsmodule.

Im zugeführten Luftstrom ist ein Gasqualitätssensor angeordnet, welcher eine Wirkschicht und eine Heizung hat. Die Heizung wird über einen elektrischen Heizungsregler so geregelt, daß die Temperatur der Wirkschicht konstant ist. Schadstoffbezogene Veränderungen der elektrischen Daten der Wirkschicht werden von einem elektrischen Steuer- und Auswertegerät ausgewertet, welches z.B. als Mikroprozessor ausgebildet ist.

Das in diesem Auswertegerät aufbereitete Sensorsignal ist bevorzugt rechnerisch von Einflüssen der Luftfeuchte, der Luftströmung und der Lufttemperatur befreit und wird als Störgröße über eine elektrische Leitung so auf den Regler aufgeschaltet, daß der Arbeitspunkt der Anordnung in Abhängigkeit vom Störgrößensignal gleiten kann.

Dies erfolgt nach der Logik:
- Mehr Luftbelastung =: Hochspannung anheben
- Gute Luft =: Hochspannung auf vorgegebenem minimalen Wert

Aus den Daten des Gasqualitätssensors können mit Hilfe geeigneter Signalverarbeitung Schaltsignale zur Beeinflussung der Arbeitsweise der lufttechnischen Anlage erzeugt werden. Zum Beispiel wird in einem Automobil die Ionisationseinrichtung hinter der Umluftklappe und vor dem Luftkühler (Verdampfer) bzw. Heizer angeordnet, um die Luft von Geruchsstoffen und Bakterien zu befreien und um die Besiedlung des Luftkühlers mit Keimen und Pilzen zu unterbinden.

Der Gasqualitätssensor wird im Luftstrom vorzugsweise unmittelbar vor den lonisationsmodulen angeordnet und hat wesentlich die erfindungsgemäße Funktion, die Ionisationsleistung der Luftqualität zu steuern. Dazu kann das Gasqualitätssensorsignal so aufbereitet werden, daß dann, wenn die Qualität der zugeführten Außenluft sich über ein bestimmtes Maß hinaus verschlechtert, ein Schaltsignal ausgelöst wird, welches die Luftklappe in die Position "Umlauf-Zirkulation" stellt.

Nunmehr ist der Sensor der rückgeführten Kabinenluft ausgesetzt. Wird die Kabinenluft durch z.B. Tabakrauch in erhöhtem und über ein festgelegtes Maß hinaus belastet, wird dies vom Gasqualitätssensor festgestellt und es wird ein Schaltimpuls erzeugt, welcher die Umluftklappe wieder auf Normalbetrieb stellt, d.h., es strömt wieder Außenluft ein.

In Automobilen wird die Luftklappe zumeist über ein Klimasteuergerät kontrolliert. Hier liegen Informationen über den Schaltzustand (Zuluft oder Umluft) vor. Erfindungsgemäß wird daher vorgeschlagen, diese Daten mit den Sensordaten zu korrelieren, so daß die Luftklappe stets in einer Position steht, die die situationsbezogen jeweils maximal erreichbare Luftqualität ermöglicht.

Vorteilhaft wird durch die beschriebene Ausformung der Erfindung erreicht, daß kostensparend die Erzeugung des Steuersignals zur Situationsanpassung der lonisatonsstärke, Störgrößenaufschaltung, und die Erzeugung von Schaltsignalen zum Zwecke der Luftführung mit einem einzigen Gasqualitätssensor erfolgt. Bei der Anwendung im Automobil löst die Erfindung das bei außenluftbasierter Steuerung der Luftführung auftretende Problem, daß sich unbemerkt in der Kabine bedenkliche Zustände der Luftqualität aufbauen können, z.B. dann, wenn in der Kabine stark geraucht wird. Dies kann vorteilhaft bei Anwendung der Erfindung nicht erfolgen, weil die Umschaltung durch Vergleich der Qualität der zuletzt zugeführten Außenluft mit der aktuellen Qualität der rückgeführten Kabinenluft erfolgt. Die Rückschaltung auf Außenluft erfolgt in diesem Fall dann, wenn die Qualität der Kabinenluft schlechter wird als die zuletzt zugeführte Außenluft.

Weiter wird erfindungsgemäß vorgeschlagen, das zum Zwecke der Störgrößenaufschaltung erzeugte und die Luftqualität abbildende Gasqualitätssensorignal in geeigneter Form zur Anzeige zu bringen. Damit kann visuell der Nutzer über die Qualität der ihm zugeführten Luft informiert werden. Wird durch interaktive Auswertung des Gasqualitätssensors das Gasqualitätssensorsignal von Einflüssen der Luftfeuchte, der Lufttemperatur und der Luftströmung befreit, wird entgegen zu den bekannten einfachen Luftqualitäts-Anzeigen (zumeist basierend auf einem Spannungsteiler, der sich aus einem Sensor und einem ohmschen Widerstand bildet) die wahre Luftqualität, also die Quantität der in der Luft anwesenden oxidierbaren Luftbestandteile zur Anzeige gebracht.

Selbstverständlich ist die Erfindung nicht auf Lüftungssysteme bzw. Klimaanlagen von Automobilen beschränkt ist, sondern kann sinngemäß auch bei der Belüftung und Klimatisierung von Gebäuden, Räumen und anderen lüftungstechnischen Anwendungen zum Einsatz kommen.

In einer bevorzugten Ausgestaltung der Erfindung befindet sich zwischen den beiden Elektroden 3, 4 mindestens ein Dielektrikum. Insbesondere kann das Gasentladungsmodul 2 eine Vorrichtung zur Erzeugung eines Plasmas zur Herstellung von Ozon und/oder Sauerstoffionen in Luft nach dem Prinzip der dielektrisch behinderten Entladung enthalten und die Elektroden 3, 4 Bestandteile einer derartigen Vorrichtung sein. Eine derartige Vorrichtung ist in der WO 01/02291 A2 beschrieben.

Gemäß einer weiteren Variante des erfindungsgemäßen Verfahrens kann der Erschöpfungsgrad eines Gasfilters, z.B. des Luftfilters 21 von Fig. 9, festgestellt werden. Fig. 10 zeigt zur Erläuterung dieses Verfahrens ein Diagramm des zeitlichen Verlaufes der Konzentrationen eines Prüfgases vor und nach Durchlaufen des Gasfilters. Erfindungsgemäß wird dem Gasstrom vor Erreichen des Filters für eine bestimmte definierte Zeitspanne, z.B. 30 Sekunden, in einer konstanten Konzentration ein Prüfgas zugesetzt. Als Prüfgas kann z.B. Cyclohexan verwendet werden.

Den Zeitverlauf des Konzentration des Prüfgases vor Erreichen des Filters zeigt Kurve 30 in Fig. 10. Die Fähigkeit des Gasfilters, das Prüfgas zu adsorbieren, nimmt mit steigender Erschöpfung des Filters ab. Nach Durchlaufen des Gasfilters passiert der Gasstrom und somit auch das vom Gasfilter nicht adsorbierte Prüfgas einen Gasqualitätssensor, der das Prüfgas zu detektieren imstande ist und ein Gasqualitätssensorsignal abgibt, das ein Maß für die Konzentration des Prüfgases im Gasstrom ist.

Erfindungsgemäß wird hierbei der Umstand ausgenutzt, daß die Steilheit des zeitlichen Anstieges des Gasqualitätssensorsignals nach Beginn und des zeitlichen Gefälles des Gasqualitätssensorsignals nach Ende des Zusetzens des Prüfgases mit dem Erschöpfungsgrad des Gasfilters abnehmen aus der Steilheit dieser Steigung bzw. dieses Gefälles bestimmt wird. Für ein Gasfilter mit geringem Erschöpfungsgrad ergibt sich z.B. die Kurve 31 von Fig. 10, für ein Gasfilter mit großem Erschöpfungsgrad z.B. die Kurve 32 von Fig. 10.

Als vorteilhaft für die Anwendung dieses Verfahrens für das Belüftungssystem bzw. die Klimaanlage erweist sich hierbei die Tatsache, daß der Gasqualitätssensor hinter dem in Automobilen zumeist vorhandenen Eingangs-Luftfilter angeordnet wird. Luftfilter haben die Eigenschaft, Gase und Dämpfe zu adsorbieren. Die adsorbierten Gase und Dämpfe werden im Anschluß zeitverzögert wieder abgegeben (desorbiert). Der Prüfgasimpuls erfährt durch einen leistungsfähigen Filter eine typische Verzerrung, wie er in sogenannten elektrischen Tiefpaßfiltern ebenfalls beobachtet wird. Der Gradient. d.h. der Anstieg der Gaskonzentration nach dem Luftfilter, ist bei erschöpften Filtern deutlich steiler als bei leistungsfähigen Filtern. Bei erschöpften Filtern ist aus diesem Grund die Signaldynamik deutlich höher als bei leistungsfähigen Filtern.

Die Signalauswertung des Gasqualitätssensors bewertet erfindungsgemäß die durchschnittliche Signaldynamik über die Zeit und gewinnt daher eine Information zur Befähigung des Filters zur temporären Adsorbtion von Gasen oder Dämpfen. Diese Information wird von der Signalauswertung des Gasqualitätssensors als Schaltsignal herausgegeben und zeigt an, daß der Eingangsluftfilter erschöpft ist und gewechselt werden sollte.

Die Lehre der beschriebenen Erfindung ist nicht auf die beispielhaft dargestellten Anwendungen beschränkt sondern kann vorteilhaft immer dann in lufttechnischen Anlagen eingesetzt werden, in denen elektrisch geregelte Gasentladungsmodule, z.B. lonisations- bzw. Ozonisationsapparate, eingesetzt werden, welche durch Gasqualitätssensors situationsadaptiert gesteuert werden, wobei die Signalauswertung der Gasqualitätssensoren zielorientiert so ausgeführt ist, so daß neben der Steuerung der Ionisationsleistung zusätzlich sinnvolle und zusätzliche Eingriffe in die Luftführung möglich werden.

### Liste der Bezugszeichen:

- 1: Hochspannungsgenerator
- 1a: Steuereingang von 1
- 2: Gasentladungsmodul
- 3, 4: Elektroden von 2
- 5: Gas
- 6: Regler
- 6a: Ist-Wert-Eingang von 6
- 7: frequenzselektives Filter
- 10a, b: Leitungen
- 11a, b: Leitungen
- 12: Mikrofon
- 13: Fotodetektor
- 14: Einlaß
- 15: Auslaß
- 16,116: Regler
- 16a, 116a: Ist-Wert-Eingang von 16,116
- 16b, 116b: Sollwert-Eingang von 16,116
- 17,117: Steuergerät
- 17a: Ausgang von 17
- 117a, b: erster, zweiter Ausgang von 117
- 18: Gasqualitätssensor
- 20: Ventilator
- 21: Gasfilter
- 22: Heizer
- 23: Kühler
- 24: Katalysator
- 25: Kabine
- 26: Antrieb
- 27: Luftklappe
- 27a: Stellung von 27a in Position "Zulauf-Ablauf-Betrieb"
- 28: Stellmechanismus für 27
R1, R2 erster, zweiter Widerstand
R3, R4 dritter, vierter Widerstand

## Patentansprüche

1. Verfahren zur Regelung der elektrischen Kapazität, welche zwischen zwei Elektroden (3, 4) eines Gasentladungsmoduls (2) besteht, wobei das Gasentladungsmodul (2) eine Vorrichtung zur Erzeugung eines Plasmas zur Herstellung von Ozon und/oder Sauerstoffionen in Luft nach dem Prinzip der dielektrisch behinderten Entladung enthält und zwischen den Elektroden (3,4) eine von einem steuerbaren Hochspannungsgenerator (1) erzeugte Hochspannung angelegt ist, die zur Auslösung einer Gasentladung in dem Gas (5) des Gasentladungsmoduls (2) dient und die entweder eine Wechselspannung oder eine gepulste Gleichspannung ist, und die Gasentladung ein Plasma erzeugt, durch welches die effektive Fläche der Elektroden (3,4) und damit die zwischen denselben bestehende Kapazität vergrößert wird, **dadurch gekennzeichnet, daß**, wenn der Ist-Wert der zwischen den beiden Elektroden (3, 4) bestehenden Kapazität unterhalb eines bestimmten Sollwertes liegt, der Effektivwert der Hochspannung des Hochspannungsgenerators (1) zeitlich zunehmend gesteuert wird, oder wenn der Ist-Wert der zwischen den beiden Elektroden (3, 4) bestehenden elektrischen Kapazität oberhalb des Sollwertes liegt, der Effektivwert der Hochspannung des Hochspannungsgenerators (1) zeitlich abnehmend gesteuert wird.

2. Verfahren nach Anspruch 1, **dadurch gekennzeichnet,**
**daß** der Ist-Wert der zwischen den beiden Elektroden (3, 4) bestehenden elektrischen Kapazität aus
- der integralen Intensität oder
- Intensität mindestens einer bestimmten Frequenz oder mindestens eines bestimmten Frequenzbereiches oder
- der mittleren Frequenz oder
- der Frequenzverteilung oder
- der Frequenz der maximalen Intensität
des statistischen elektrischen Rauschens, mit welchem der durch die Hochspannung in Gang gesetzte elektrische Strom aufgrund der Gasentladung behaftet ist, bestimmt oder abgeleitet wird.

3. Verfahren nach Anspruch 2, **dadurch gekennzeichnet,**
**daß** das statistische elektrische Rauschen erfaßt wird, indem der Strom über einen zweiten Widerstand (R2) oder eine Induktivität geführt wird, von welchem bzw. welcher das statistische elektrische Rauschen über ein als Bandpaß oder als Hochpaß fungierendes frequenzselektives Filter (7) abgegriffen wird.

4. Verfahren nach einem der Ansprüche 1 bis 3, **dadurch gekennzeichnet,**
**daß** der Ist-Wert der zwischen den beiden Elektroden (3, 4) bestehenden elektrischen Kapazität aus
- der integralen Intensität oder
- Intensität mindestens einer bestimmten Frequenz oder mindestens eines bestimmten Frequenzbereiches oder
- der mittleren Frequenz oder
- der Frequenzverteilung oder
- der Frequenz der maximalen Intensität
des statistischen akustischen Rauschens, welches durch die Gasentladung in dem Gas (5) erzeugt wird, bestimmt oder abgeleitet wird.

5. Verfahren nach Anspruch 4, **dadurch gekennzeichnet,**
**daß** das statistische akustische Rauschen mittels eines akustischen Wandlers, insbesondere Mikrofon (12), erfaßt wird.

6. Verfahren nach einem der Ansprüche 1 bis 5, **dadurch gekennzeichnet,**
**daß** der Ist-Wert der zwischen den beiden Elektroden (3, 4) bestehenden elektrischen Kapazität aus
- der integralen Intensität oder
- Intensität mindestens einer bestimmten Wellenlänge oder Wellenlängenbereiches oder
- der mittleren Wellenlänge oder
- der spektralen Wellenlängenverteilung oder
- der Wellenlänge der maximalen Intensität
von elektromagnetischer Strahlung, z.B. Licht, Röntgenstrahlung, Radiostrahlung, die durch die Gasentladung erzeugt und abgestrahlt wird, bestimmt oder abgeleitet wird.

7. Verfahren nach einem der Ansprüche 1 bis 6, **dadurch gekennzeichnet,**
**daß** der Ist-Wert der zwischen den beiden Elektroden (3, 4) bestehenden elektrischen Kapazität aus der Phasenverschiebung zwischen der Hochspannung und dem durch diese in Gang gesetzten elektrischen Strom bestimmt oder abgeleitet wird.

8. Verfahren nach einem der Ansprüche 1 bis 7, **dadurch gekennzeichnet,**
**daß** der Ist-Wert der zwischen den beiden Elektroden (3, 4) bestehenden elektrischen Kapazität aus der Größe des durch die Hochspannung in Gang gesetzten elektrischen Stromes bestimmt oder abgeleitet wird.

9. Verfahren nach Anspruch 8, **dadurch gekennzeichnet,**
**daß** der elektrische Strom bestimmt wird, indem er über einen ersten Widerstand (R1) geführt und die an diesem abfallende Spannung abgegriffen und als Maß für den durch die Hochspannung in Gang gesetzten elektrischen Strom herangezogen wird.

10. Verfahren nach einem der Ansprüche 1-9, **dadurch gekennzeichnet,**
**daß** der Ist-Wert und der Sollwert der zwischen den beiden Elektroden (3, 4) bestehenden elektrischen Kapazität mittels eines Reglers (6), z.B. PID-Regler, verglichen werden, wobei der Regler (6) aus der Differenz zwischen Ist-Wert und Sollwert oder dem zeitlichen Verhalten dieser Differenz ein zeitlich veränderliches Stellsignal bildet, welches zur Steuerung des Hochspannungsgenerators (1) herangezogen wird.

11. Verfahren nach einem der Ansprüche 1-10, **dadurch gekennzeichnet,**
**daß** es sich bei dem Gas (5) um Luft handelt und der Sollwert so gewählt ist, daß bei der Gasentladung Ozon und Sauerstoff-Ionen, jedoch kein Stickstoffoxid NOx entsteht.

12. Verfahren nach Anspruch 1, **dadurch gekennzeichnet,**
**daß** die Wellenform der Wechselspannung oder der gepulsten Gleichspannung sinusförmig oder im wesentlichen rechteckförmig ist.

13. Verfahren nach Anspruch 1, **dadurch gekennzeichnet,**
**daß** die Zeitkonstante der Regelung kürzer ist als die halbe Periode der Wechselspannung oder der gepulsten Gleichspannung, so daß durch die Regelung die Wellenform der Wechselspannung oder der gepulsten Gleichspannung beeinflußt wird.

14. Verfahren nach einem der Ansprüche 1 bis 13, **dadurch gekennzeichnet,**
**daß** die Frequenz der Wechselspannung oder der gepulsten Gleichspannung zwischen 15 kHz und 35 kHz beträgt.

15. Verfahren nach einem der Ansprüche 1 bis 4, **dadurch gekennzeichnet,**
**daß** die Vorrichtung zur Erzeugung eines Plasmas zur Herstellung von Ozon und/oder Sauerstoffionen in Luft nach dem Prinzip der dielektrisch behinderten Entladung folgende Komponenten umfaßt:
a) einen flachen, elektrisch isolierenden Träger, dessen Material eine Dielektrizitätskonstante von wenigstens größer 30 aufweist,
b) eine Elektrode (3), untere Elektrode (3), die auf der einen Hauptoberfläche des Trägers, Rückseite, aus einem elektrisch leitfähigen Material aufgebracht ist,
c) wenigstens eine elektrische Isolierschicht aus einem dielektrischen Material, die auf der anderen, der Luft ausgesetzten Hauptoberfläche des Trägers, Vorderseite, aufgebracht ist, wobei die Isolierschicht die Vorderseite des Trägers nur teilweise bedeckt, und die Dielektrizitätskonstante des Trägers und diejenige der Isolierschicht unterschiedlich sind, wobei die Differenz zwischen der Dielektrizitätskonstante des Trägers und der Isolierschicht bzw. der Teilschichten so gewählt ist, dass sich der Effekt der Spiegelentladungen einstellt,
d) eine auf der Isolierschicht angeordnete Elektrode (4), obere Elektrode, aus einem elektrisch leitfähigen Material, welche die Isolierschicht nur teilweise bedeckt, wobei an die beiden Elektroden (3, 4) eine Hochspannung des Hochspannungsgenerators (1) gelegt ist.

16. Verfahren nach einem der Ansprüche 1-15, **dadurch gekennzeichnet,**
**daß** das Gas (5) mittels einer Fördereinrichtung, z.B. Ventilator (20), so gefördert wird, daß es das Gasentladungsmodul (2) durchströmt.

17. Verfahren nach Anspruch 16, **dadurch gekennzeichnet,**
**daß** in dem Gasstrom ein Gasqualitätssensor (18) so angeordnet ist, daß das Gas (5) den Gasqualitätssensor (18) vor Erreichen des Gasentladungsmoduls (2) passiert, wobei der Gasqualitätssensor (18) ein Gasqualitätssensorsignal abgibt, welches ein Maß für die Belastung des Gases (5) mit Fremdstoffen, Schadstoffen oder Keimen ist und dergestalt zur Beeinflussung des Sollwertes herangezogen wird, daß der Sollwert mit zunehmender bzw. abnehmender Belastung des Gases (5) mit Fremdstoffen, Schadstoffen oder Keimen angehoben bzw. abgesenkt wird.

18. Verfahren nach Anspruch 1, **dadurch gekennzeichnet,**
**daß** sich die Elektroden (3,4) in einem strömenden Gas (5), insbesondere Luft, befinden, und zur Regelung der Ausgangsspannung des Hochspannungsgenerators (1) ein Regler (6, 26) den Spannungs-Ist-Wert mit einem Spannungs-Sollwert vergleicht und, wenn der Spannungs-Ist-Wert unterhalb des Spannungs-Sollwertes liegt, die Hochspannung zeitlich zunehmend gesteuert wird, oder wenn der Spannungs-Ist-Wert oberhalb des Spannungs-Sollwertes liegt, die Hochspannung zeitlich abnehmend gesteuert wird, wobei in dem Gasstrom ein Gasqualitätssensor (18) so angeordnet ist, daß das Gas (5) den Gasqualitätssensor (18) vor Erreichen der Elektroden (3, 4) passiert, wobei der Gasqualitätssensor (18) ein Gasqualitätssensorsignal abgibt, welches ein Maß für die Belastung des Gases (5) mit Fremdstoffen, Schadstoffen oder Keimen ist und dergestalt zur Beeinflussung des Spannungs-Sollwertes herangezogen wird, daß der Spannungs-Sollwert mit zunehmender bzw. abnehmender Belastung des Gases (5) mit Fremdstoffen, Schadstoffen oder Keimen angehoben bzw. abgesenkt wird.

19. Verfahren nach Anspruch 1, **dadurch gekennzeichnet,**
**daß** sich die Elektroden (3,4) in einem strömenden Gas (5), insbesondere Luft, befinden, und zur Regelung der Ionisisierungsleistung der Gasentladung die Hochspannung über einen dritten Widerstand (R3) zwischen den Elektroden (3, 4) angelegt ist und der Ionisierungsleistungs-Ist-Wert aus dem Wert der Hochspannung und der am dritten Widerstand (R3) abfallenden Spannung ermittelt wird und ein Regler (6, 16) den Ionisierungsleistungs-Ist-Wert mit einem Ionisierungsleistungs-Sollwert vergleicht und, wenn der Ionisierungsleistungs-Ist-Wert unterhalb des Ionisierungsleistungs-Sollwertes liegt, die Hochspannung zeitlich zunehmend gesteuert wird, oder wenn der Ionisierungsleistungs-Ist-Wert oberhalb des Ionisierungsleistungs-Sollwertes liegt, die Hochspannung zeitlich abnehmend gesteuert wird, wobei in dem Gasstrom ein Gasqualitätssensor (18) so angeordnet ist, daß das Gas (5) den Gasqualitätssensor (18) vor Erreichen der Elektroden passiert, wobei der Gasqualitätssensor (18) ein Gasqualitätssensorsignal abgibt, welches ein Maß für die Belastung des Gases (5) mit Fremdstoffen, Schadstoffen oder Keimen ist und dergestalt zur Beeinflussung des Spannungs-Sollwertes herangezogen wird, daß der Ionisierungsleistungs-Sollwert mit zunehmender bzw. abnehmender Belastung des Gases (5) mit Fremdstoffen, Schadstoffen oder Keimen angehoben bzw. abgesenkt wird.

20. Verfahren nach einem der Ansprüche 17 bis 19, **dadurch gekennzeichnet,**
**daß** der Gasqualitätssensor (18) ein Sensor für Gerüche oder Verkeimung ist.

21. Verfahren nach einem der Ansprüche 17 bis 19, **dadurch gekennzeichnet,**
**daß** der Gasqualitätssensor (18) ein Sensor für gasförmige oder partikelförmige Fremd- oder Schadstoffe ist.

22. Verfahren nach einem der Ansprüche 17 bis 19, **dadurch gekennzeichnet,**
**daß** das Gas (5) Luft ist und der Gasqualitätssensor (18) ein Sensor für oxidierbare in der Luft enthaltene gasförmige oder partikelförmige Fremd- oder Schadstoffe ist.

23. Verfahren nach einem der Ansprüche 17 bis 22,
**dadurch gekennzeichnet, daß** das Gasqualitätssensorsignal dazu herangezogen wird, das Gas (5) in einen Zulauf-Ablauf-Betrieb zu versetzen, in welchem es dem Gasqualitätssensor (18) von außen zugeführt wird und nach Passieren der Elektroden (3, 4) wieder nach außen abgeführt wird, wenn die Konzentration von Fremdstoffen, Schadstoffen oder Keimen in dem Gas (5) einen bestimmten Grenzwert übersteigt bzw. unterschreitet, oder das Gas (5) in eine Umlauf-Zirkulation zu versetzen, in welcher das Gas (5) nach Passieren der Elektroden (3, 4) in einem geschlossenen Kreislauf erneut an den Gasqualitätssensor (18) geführt wird, wenn die Konzentration von Fremdstoffen, Schadstoffen oder Keimen in dem Gas (5) einen bestimmten Grenzwert unterschreitet bzw. übersteigt.

24. Verfahren nach Anspruch 23, **dadurch gekennzeichnet,**
**daß**, wenn die Konzentration von Fremdstoffen, Schadstoffen oder Keimen in dem Gas (5), nachdem das Gas (5) in die Umlauf-Zirkluation bzw. in den Zulauf-Ablauf-Betrieb versetzt wurde, weiterhin ansteigt, das Gas (5) wieder zurück in den Zulauf-Ablauf-Betrieb bzw. zurück in die Umlauf-Zirkulation versetzt wird.

25. Verfahren nach Anspruch 23, **dadurch gekennzeichnet,**
**daß** das Gas (5) bei Zulauf-Ablauf-Betrieb nach einer bestimmten Zeit in die Umlauf-Zirkulation versetzt wird, wobei der Gasqualitätssensor (18) unmittelbar vor dem Übergang in die Umlauf-Zirkulation einen ersten Meßwert und unmittelbar nach dem Übergang einen zweiten Meßwert für die Konzentration von Fremdstoffen, Schadstoffen oder Keimen liefert, wobei das Gas (5) nur dann wieder in den Zulauf-Ablauf-Betrieb zurückversetzt wird, wenn der erste Meßwert niedriger ist als der zweite Meßwert, oder
**daß** das Gas (5) bei Umlauf-Zirkulation nach der bestimmten Zeit in den Zulauf-Ablauf-Betrieb versetzt wird, wobei der Gasqualitätssensor (18) unmittelbar vor dem Übergang in den Zulauf-Ablauf-Betrieb einen dritten Meßwert und unmittelbar nach dem Übergang einen vierten Meßwert für die Konzentration von Fremdstoffen, Schadstoffen oder Keimen liefert, wobei das Gas (5) nur dann wieder in die Umlauf-Zirkulation zurückversetzt wird, wenn der dritte Meßwert niedriger ist als der vierte Meßwert.

26. Verfahren nach Anspruch 25, **dadurch gekennzeichnet,**
**daß** der erste und der zweite Meßwert miteinander verglichen werden und als Grenzwert der niedrigere dieser beiden Meßwerte übernommen wird, bzw. daß der dritte und der vierte Meßwert miteinander verglichen werden und als Grenzwert der niedrigere dieser beiden Meßwerte übernommen wird, so daß der Grenzwert in den bestimmten Zeitabständen durch einen neuen Grenzwert ersetzt wird.

27. Verfahren nach einem der Ansprüche 16 bis 26,
**dadurch gekennzeichnet, daß** das Gasqualitätssensorsignal dazu herangezogen wird, mit zunehmender Konzentration von Fremdstoffen, Schadstoffen oder Keimen in dem Gas die Strömungsgeschwindigkeit des Gases (5) zu vergrößern und umgekehrt.

28. Verfahren nach einem der Ansprüche 17 bis 27,
**dadurch gekennzeichnet, daß** die Störeinflüsse von Luftfeuchte, Strömungsgeschwindigkeit und Gastemperatur auf das Gasqualitätssensorsignal rechnerisch kompensiert werden.

29. Verfahren nachAnspruch 28, **dadurch gekennzeichnet,**
**daß** die Konzentration von Fremdstoffen, Schadstoffen oder Keimen nach der Kompensation der Störeinflüsse von Luftfeuchte, Strömungsgeschwindigkeit und Gastemperatur auf einem Display angezeigt wird.

30. Verfahren nach einem der Ansprüche 16 bis 29, **dadurch gekennzeichnet,**
**dass** im Gasstrom ein Gasfilter (21), z.B. Luftfilter, angeordnet ist, dessen Fähigkeit, ein dem Gasstrom zugesetztes Prüfgas zu adsorbieren, mit steigender Erschöpfung des Gasfilters (21) abnimmt, wobei
- der Gasstrom nach Durchlaufen des Gasfilters (21) einen Gasqualitätssensor (18) passiert, welcher das Prüfgas zu detektieren imstande ist und ein Gasqualitätssensorsignal abgibt, das ein Maß für die Konzentration des Prüfgases ist,
- das Prüfgas dem Gasstrom vor Erreichen des Gasfilters (21) für eine bestimmte definierte Zeitspanne in einer konstanten Konzentration zugesetzt wird,
- und der Erschöpfungsgrad des Gasfilters (21) unter Ausnutzung des Umstandes, daß die Steilheit des zeitlichen Anstieges des Gasqualitätssensorsignals nach Beginn und des zeitlichen Gefälles des Gasqualitätssensorsignals nach Ende des Zusetzens des Prüfgases mit dem Erschöpfungsgrad des Gasfilters (21) abnehmen, aus der Steilheit dieser Steigung bzw. dieses Gefälles bestimmt wird.

31. Vorrichtung zur Erzeugung einer Gasentladung, umfassend ein Gasentladungsmodul (2) mit zwei in einem Gas (5), insbesondere Luft, befindlichen Elektroden (3,4), und einen steuerbaren Hochspannungsgenerator (1), welcher entweder eine Wechselspannung oder eine gepulste Gleichspannung abgibt,
wobei das Gasentladungsmodul (2) eine Vorrichtung zur Erzeugung eines Plasmas zur Herstellung von Ozon und/oder Sauerstoffionen in Luft nach dem Prinzip der dielektrisch behinderten Entladung enthält, und die Elektroden (3,4) so an den Hochspannungsgenerator (1) angeschlossen sind, daß die Wechselspannung oder die gepulste Gleichspannung zwischen den Elektroden (3, 4) anliegt, und die Gasentladung ein Plasma erzeugt, durch welches die effektive Fläche der Elektroden (3,4) und damit die zwischen denselben bestehende Kapazität vergrößert wird, **dadurch gekennzeichnet,**
**daß** die Vorrichtung ferner einen Regler (6) umfaßt, welcher den Ist-Wert der zwischen den beiden Elektroden (3, 4) bestehenden Kapazität oder einen hieraus abgeleiteten Wert mit einem vorgebbaren Sollwert vergleicht,
wobei, wenn der Ist-Wert der zwischen den beiden Elektroden (3, 4) bestehenden Kapazität unterhalb eines bestimmten Sollwertes liegt, der Effektivwert der Hochspannung des Hochspannungsgenerators (1) durch den Regler (6) zeitlich zunehmend gesteuert wird,
oder wenn der Ist-Wert der zwischen den beiden Elektroden (3, 4) bestehenden elektrischen Kapazität oberhalb des Sollwertes liegt, der Effektivwert der Hochspannung des Hochspannungsgenerators (1) durch den Regler (6) zeitlich abnehmend gesteuert wird.

32. Vorrichtung nach Anspruch 31, **dadurch gekennzeichnet,**
**daß** ein Rauschsensor das statistische elektrische Rauschen, mit welchem der durch die Hochspannung in Gang gesetzte Strom aufgrund der Gasentladung behaftet ist, erfaßt und in ein zweites Sensorsignal umsetzt und der Ist-Wert der zwischen den beiden Elektroden (3, 4) bestehenden elektrischen Kapazität aus dem zweiten Sensorsignal bestimmt wird.

33. Vorrichtung nach Anspruch 32, **dadurch gekennzeichnet,**
**daß** der Rauschsensor umfaßt:
- einen zweiten Widerstand (11) oder eine Induktivität, über den bzw. über die der durch die Hochspannung in Gang gesetzte elektrische Strom geführt ist, und
- ein als Bandpaß oder als Hochpaß fungierendes frequenzselektives elektrisches Filter (12),
wobei die an dem zweiten Widerstand (11) bzw. an der Induktivität abfallende Spannung abgegriffen wird und nach Passieren des frequenzselektiven elektrischen Filters (12) als zweites Sensorsignal dient.

34. Vorrichtung nach einem der Ansprüche 31 bis 33, **dadurch gekennzeichnet,**
**daß** ein akustischer Sensor, insbesondere Mikrofon (12), das akustische Rauschen, welches durch die Gasentladung in dem Gas erzeugt wird, erfaßt und in ein drittes Sensorsignal umsetzt und der Ist-Wert der zwischen den beiden Elektroden (3, 4) bestehenden elektrischen Kapazität aus dem dritten Sensorsignal bestimmt wird.

35. Vorrichtung nach einem der Ansprüche 31 bis 34, **dadurch gekennzeichnet,**
**daß** ein Strahlungssensor (13) elektromagnetische Strahlung, wie Licht, Röntgenstrahlung oder Radiostrahlung, die durch die Gasentladung erzeugt und abgestrahlt wird, erfaßt und in ein viertes Sensorsignal umsetzt und der Ist-Wert der zwischen den beiden Elektroden (3, 4) bestehenden elektrischen Kapazität aus dem vierten Sensorsignal bestimmt wird.

36. Vorrichtung nach einem der Ansprüche 31 bis 35, **dadurch gekennzeichnet,**
**daß** ein Phasenvergleicher die Phasenverschiebung zwischen der Hochspannung und dem durch diese in Gang gesetzten elektrischen Strom in ein fünftes Sensorsignal erfaßt und in ein fünftes Sensorsignal umsetzt, und der Ist-Wert der zwischen den beiden Elektroden (3, 4) bestehenden elektrischen Kapazität aus dem fünften Sensorsignal bestimmt oder abgeleitet wird.

37. Vorrichtung nach einem der Ansprüche 31 bis 36, **dadurch gekennzeichnet,**
**daß** ein Stromsensor den durch die Hochspannung in Gang gesetzten elektrischen Strom erfaßt und in ein erstes Sensorsignal umsetzt und der Ist-Wert der zwischen den beiden Elektroden bestehenden elektrischen Kapazität aus dem ersten Sensorsignal bestimmt wird.

38. Vorrichtung nach Anspruch 37, **dadurch gekennzeichnet,**
**daß** der Stromsensor ein erster Widerstand (10) ist, über den der durch die Hochspannung in Gang gesetzte elektrische Strom geführt wird, wobei die an dem ersten Widerstand (10) abfallende Spannung abgegriffen und als erstes Sensorsignal dient.

39. Vorrichtung nach Anspruch 31, **dadurch gekennzeichnet,**
**daß** die Wellenform der Wechselspannung oder der gepulsten Gleichspannung sinusförmig oder im wesentlichen rechteckförmig ist.

40. Vorrichtung nach Anspruch 31, **dadurch gekennzeichnet,**
**daß** die Zeitkonstante der Regelung kürzer ist als die halbe Periode der Wechselspannung oder der gepulsten Gleichspannung, so daß durch die Regelung die Wellenform der Wechselspannung oder der gepulsten Gleichspannung beeinflußt wird.

41. Vorrichtung nach einem der Ansprüche 31 bis 40, **dadurch gekennzeichnet,**
**daß** die Frequenz der Wechselspannung oder der gepulsten Gleichspannung zwischen 15 kHz und 35 kHz beträgt.

42. Vorrichtung nach Anspruch 31, **dadurch gekennzeichnet,**
**daß** der Regler (6) ein PID-Regler ist.

43. Vorrichtung nach einem der Ansprüche 31-42, **dadurch gekennzeichnet,**
**daß** es sich bei dem Gas (5) um Luft handelt und der Sollwert so gewählt ist, daß bei der Gasentladung Ozon und Sauerstoff-Ionen, jedoch kein Stickstoffoxid entsteht.

44. Vorrichtung nach einem der Ansprüche 31 bis 33, **dadurch gekennzeichnet,**
**daß** die Vorrichtung zur Erzeugung eines Plasmas zur Herstellung von Ozon und/oder Sauerstoffionen in Luft nach dem Prinzip der dielektrisch behinderten Entladung folgende Komponenten umfaßt:
a) einen flachen, elektrisch isolierenden Träger, dessen Material eine Dielektrizitätskonstante von wenigstens größer 30 aufweist,
b) eine Elektrode (3), untere Elektrode (3), die auf der einen Hauptoberfläche des Trägers, Rückseite, aus einem elektrisch leitfähigen Material aufgebracht ist,
c) wenigstens eine elektrische Isolierschicht aus einem dielektrischen Material, die auf der anderen, der Luft ausgesetzten Hauptoberfläche des Trägers, Vorderseite, aufgebracht ist, wobei die Isolierschicht die Vorderseite des Trägers nur teilweise bedeckt, und die Dielektrizitätskonstante des Trägers und diejenige der Isolierschicht unterschiedlich sind, wobei die Differenz zwischen der Dielektrizitätskonstante des Trägers und der Isolierschicht bzw. der Teilschichten so gewählt ist, dass sich der Effekt der Spiegelentladungen einstellt,
d) eine auf der Isolierschicht angeordnete Elektrode (4), obere Elektrode (4), aus einem elektrisch leitfähigen Material, welche die Isolierschicht nur teilweise bedeckt,
wobei an die beiden Elektroden (3, 4) eine Hochspannung des Hochspannungsgenerators (1) gelegt ist.

45. Vorrichtung nach einem der Ansprüche 31-44, **dadurch gekennzeichnet,**
**daß** das Gas (5) durch eine Fördereinrichtung in Strömung versetzt wird und in dem Gasstrom ein Gasqualitätssensor (18) so angeordnet ist, daß das Gas (5) den Gasqualitätssensor (18) vor Erreichen des Gasentladungsmoduls (2) passiert,
wobei der Gasqualitätssensor (18) ein Gasqualitätssensorsignal abgibt, welches ein Maß für die Belastung des Gases (5) mit Fremdstoffen, Schadstoffen oder Keimen ist und dergestalt zur Beeinflussung des Sollwertes herangezogen wird, daß der Sollwert mit zunehmender bzw. abnehmender Belastung des Gases (5) mit Fremdstoffen, Schadstoffen oder Keimen angehoben bzw. abgesenkt wird.

46. Vorrichtung nach Anspruch 45, **dadurch gekennzeichnet,**
**daß** zu jeder Konzentration von Fremdstoffen, Schadstoffen oder Keimen in dem Gas (5) in umkehrbar-eindeutiger Zuordnung ein bestimmter Sollwert gehört.

47. Vorrichtung nach Anspruch 45, **dadurch gekennzeichnet,**
**daß** das Gasqualitätssensorsignal dazu herangezogen wird, das Gas (5) in einen Zulauf-Ablauf-Betrieb zu versetzen, in welchem es dem Gasqualitätssensor (18) von außen zugeführt wird und nach Passieren der Elektroden (3, 4) wieder nach außen abgeführt wird, wenn die Konzentration von Fremdstoffen, Schadstoffen oder Keimen in dem Gas (5) einen bestimmten Grenzwert übersteigt bzw. unterschreitet, oder das Gas (5) in eine Umlauf-Zirkulation zu versetzen, in welcher das Gas (5) nach Passieren der Elektroden (3, 4) in einem geschlossenen Kreislauf erneut an den Gasqualitätssensor (18) geführt wird, wenn die Konzentration von Fremdstoffen, Schadstoffen oder Keimen in dem Gas (5) einen bestimmten Grenzwert unterschreitet bzw. übersteigt.

48. Vorrichtung nach Anspruch 47, **dadurch gekennzeichnet,**
**daß** das Gas (5) bei Zulauf-Ablauf-Betrieb nach einer bestimmten Zeit in die Umlauf-Zirkulation versetzt wird, wobei der Gasqualitätssensor (18) unmittelbar vor dem Übergang in die Umlauf-Zirkulation einen ersten Meßwert und unmittelbar nach dem Übergang einen zweiten Meßwert für die Konzentration von Fremdstoffen, Schadstoffen oder Keimen liefert, wobei das Gas (5) nur dann wieder in den Zulauf-Ablauf-Betrieb zurückversetzt wird, wenn der erste Meßwert niedriger ist als der zweite Meßwert, oder
**daß** das Gas (5) bei Umlauf-Zirkulation nach der bestimmten Zeit in den Zulauf-Ablauf-Betrieb versetzt wird, wobei der Gasqualitätssensor (18) unmittelbar vor dem Übergang in den Zulauf-Ablauf-Betrieb einen dritten Meßwert und unmittelbar nach dem Übergang einen vierten Meßwert für die Konzentration von Fremdstoffen, Schadstoffen oder Keimen liefert, wobei das Gas (5) nur dann wieder in die Umlauf-Zirkulation zurückversetzt wird, wenn der dritte Meßwert niedriger ist als der vierte Meßwert.

49. Vorrichtung nach Anspruch 48, **dadurch gekennzeichnet,**
**daß** der erste und der zweite Meßwert miteinander verglichen werden und als Grenzwert der niedrigere dieser beiden Meßwerte übernommen wird, bzw. daß der dritte und der vierte Meßwert miteinander verglichen werden und als Grenzwert der niedrigere dieser beiden Meßwerte übernommen wird, so daß der Grenzwert in den bestimmten Zeitabständen durch einen neuen Grenzwert ersetzt wird.

## Claims

1. A method for regulating the electric capacitance that exists between two electrodes (3, 4) of a gas discharge module (2), whereby the gas discharge module (2) has a device for generating a plasma for the production of ozone and/or oxygen ions in the air according to the principle of a dielectrically hindered discharge and a high voltage that is generated by a controllable high-voltage generator (1) is applied between the electrodes (3, 4), said high voltage serving to trigger a gas discharge in the gas (5) of the gas discharge module (2) and being either an alternating voltage or a pulsed direct voltage, and the gas discharge generates a plasma, thereby enlarging the effective surface area of the electrodes (3, 4) and thus the capacitance that exists between the two electrodes (3, 4), **characterized in that**,
when the actual value of the capacitance that exists between the two electrodes (3, 4) lies below a certain setpoint, the effective value of the high voltage of the high voltage generator (1) is controlled so as to increase over time, or when the actual value of the electric capacitance that exists between the two electrodes (3, 4) lies above the setpoint, the effective value of the high voltage of the high voltage generator (1) is controlled so as to decrease over time.

2. The method according to Claim 1, **characterized in that**
the actual value of the electric capacitance that exists between the two electrodes (3, 4) is determined or derived from
• the integral intensity or
• the intensity of at least a certain frequency or of at least a certain frequency range or
• the mean frequency or
• the frequency distribution or
• the frequency of the maximum intensity
of the statistical electric noise present in the electric current that is initiated by the high voltage as a result of the gas discharge.

3. The method according to Claim 2, **characterized in that**
the statistical electric noise is detected **in that** the current is fed through a second resistor (R2) or through an inductor from which the statistical electric noise is picked up through a frequency-selective filter (7) that functions as a band pass or as a high pass.

4. The method according to any of Claims 1 to 3, **characterized in that**
the actual value of the electric capacitance that exists between the two electrodes (3, 4) is determined or derived from
• the integral intensity or
• the intensity of at least a certain frequency or of at least a certain frequency range or
• the mean frequency or
• the frequency distribution or
• the frequency of the maximum intensity
of the statistical acoustic noise that is generated by the gas discharge in the gas (5).

5. The method according to Claim 4, **characterized in that**
the statistical acoustic noise is detected by means of an acoustic transducer, especially a microphone (12).

6. The method according to any of Claims 1 to 5, **characterized in that**
the actual value of the electric capacitance that exists between the two electrodes (3, 4) is determined or derived from
• the integral intensity or
• the intensity of at least a certain wavelength or wavelength range or
• the mean wavelength or
• the spectral wavelength distribution or
• the wavelength of the maximum intensity
of electromagnetic radiation, e.g. light, X-rays, radio waves, that is generated and emitted by the gas discharge.

7. The method according to any of Claims 1 to 6, **characterized in that**
the actual value of the electric capacitance that exists between the two electrodes (3, 4) is determined or derived from the phase shift between the high voltage and the electric current that is initiated by the high voltage.

8. The method according to any of Claims 1 to 7, **characterized in that**
the actual value of the electric capacitance that exists between the two electrodes (3, 4) is determined or derived from the magnitude of the electric current that is initiated by the high voltage.

9. The method according to Claim 8, **characterized in that**
the electric current is detected **in that** it is fed through a first resistor (R1) and the voltage dropping at this resistor is picked up and utilized as a measure of the electric current that is initiated by the high voltage.

10. The method according to any of Claims 1 to 9, **characterized in that**
the actual value and the setpoint of the electric capacitance that exists between the two electrodes (3, 4) are compared by means of a controller (6), for example, a PID controller, whereby, on the basis of the difference between the actual value and the setpoint or the behavior of this difference over time, the controller (6) forms an actuating signal that is variable over time and that is used to control the high-voltage generator (1).

11. The method according to any of Claims 1 to 10, **characterized in that**
the gas (5) is air and the setpoint is selected in such a way that ozone and oxygen ions form but no nitrogen oxide NOx forms during the gas discharge.

12. The method according to Claim 1, **characterized in that**
the waveform of the alternating voltage or of the pulsed direct voltage is sinusoidal or essentially square.

13. The method according to Claim 1, **characterized in that**
the time constant of the regulation is shorter than the half-period of the alternating voltage or of the pulsed direct voltage, so that the waveform of the alternating voltage or of the pulsed direct voltage is influenced by the regulation.

14. The method according to any of Claims 1 to 13, **characterized in that**
the frequency of the alternating voltage or of the pulsed direct voltage is between 15 kHz and 35 kHz.

15. The method according to any of Claims 1 to 4, **characterized in that**
the device for generating a plasma for the production of ozone and/or oxygen ions in the air according to the principle of a dielectrically hindered discharge comprises the following components:
a) a flat, electrically insulating carrier, whose material has a dielectric constant that is at least greater than 30,
b) an electrode (3), namely, the lower electrode (3), made of an electrically conductive material, that is applied onto one of the main surfaces of the carrier, namely, the rear,
c) at least one electric insulating layer made of a dielectric material that is applied onto the other main surface of the carrier, namely, the front, which is exposed to the air, whereby the insulating layer only partially covers the front of the carrier, and the dielectric constant of the carrier and that of the insulating layer are different, whereby the difference between the dielectric constants of the carrier and of the insulating layer or of the partial layers is selected in such a way that the mirror discharge effect occurs,
d) an electrode (4), namely, the upper electrode, made of an electrically conductive material that only partially covers the insulating layer, is situated on the insulating layer, whereby a high voltage from the high voltage generator (1) is applied to the two electrodes (3, 4).

16. The method according to any of Claims 1 to 15, **characterized in that**
the gas (5) is conveyed by means of a conveying device, for example, a fan (20), in such a way that it flows through the gas discharge module (2).

17. The method according to Claim 16, **characterized in that**
a gas quality sensor (18) is arranged in the gas stream in such a way that the gas (5) passes the gas quality sensor (18) before reaching the gas discharge module (2), whereby the gas quality sensor (18) emits a gas quality sensor signal that is a measure of the loading of the gas (5) with foreign matter, pollutants or germs and it is employed to influence the setpoint in such a way that the setpoint is raised or lowered as the loading of the gas (5) with foreign matter, pollutants or germs increases or decreases, respectively.

18. The method according to Claim 1, **characterized in that**
the electrodes (3, 4) are located in a flowing gas (5), especially air, and in order to regulate the output voltage of the high-voltage generator (1), a controller (6, 16) compares the actual value of the voltage to a voltage setpoint and, when the actual value of the voltage lies below the voltage setpoint, then the high voltage is controlled so as to increase over time, or when the actual value of the voltage lies above the voltage setpoint, the high voltage is controlled so as to decrease over time, whereby a gas quality sensor (18) is arranged in the gas stream in such a way that the gas (5) passes the gas quality sensor (18) before reaching the electrodes (3, 4), whereby the gas quality sensor (18) emits a gas quality sensor signal that is a measure of the loading of the gas (5) with foreign matter, pollutants or germs and it is employed to influence the voltage setpoint in such a way that the voltage setpoint is raised or lowered as the loading of the gas (5) with foreign matter, pollutants or germs increases or decreases, respectively.

19. The method according to Claim 1, **characterized in that**
the electrodes (3, 4) are located in a flowing gas (5), especially air, and in order to regulate the ionization power of the gas discharge, the high voltage is applied through a third resistor (R3) located between the electrodes (3, 4) and the actual value of the ionization power is determined from the value of the high voltage and from the voltage dropping at the third resistor (R3), and a controller (6, 16) compares the actual value of the ionization power to an ionization power setpoint and, when the actual value of the ionization power lies below the ionization power setpoint, the high voltage is controlled so as to increase over time or, when the ionization power setpoint lies above the ionization power setpoint, the high voltage is controlled so as to decrease over time, whereby a gas quality sensor (18) is arranged in the gas stream in such a way that the gas (5) passes the gas quality sensor (18) before reaching the electrodes, whereby the gas quality sensor (18) emits a gas quality sensor signal that is a measure of the loading of the gas (5) with foreign matter, pollutants or germs and it is employed to influence the voltage setpoint in such a way that the voltage setpoint is raised or lowered as the loading of the gas (5) with foreign matter, pollutants or germs increases or decreases, respectively.

20. The method according to any of Claims 17 to 19,
**characterized in that** the gas quality sensor (18) is a sensor for odors or for contamination with germs.

21. The method according to any of Claims 17 to 19,
**characterized in that** the gas quality sensor (18) is a sensor for gaseous or particulate foreign matter or pollutants.

22. The method according to any of Claims 17 to 19,
**characterized in that** the gas (5) is air and the gas quality sensor (18) is a sensor for oxidizable gaseous or particulate foreign matter or pollutants present in the air.

23. The method according to any of Claims 17 to 22,
**characterized in that** the gas quality sensor signal is used to put the gas (5) into a feed-discharge mode of operation in which it is supplied to the gas quality sensor (18) from the outside and, after passing the electrodes (3, 4), it is discharged again to the outside when the concentration of foreign matter, pollutants or germs in the gas (5) has exceeded or fallen below a certain limit value, or else is used to put the gas (5) into a circulating mode of operation in which the gas (5), after passing the electrodes (3, 4), is again fed in a closed circuit to the gas quality sensor (18) when the concentration of foreign matter, pollutants or germs in the gas (5) has exceeded or fallen below a certain limit value.

24. The method according to Claim 23, **characterized in that**,
when the concentration of foreign matter, pollutants or germs in the gas (5) continues to increase after the gas (5) has been put into the circulating mode of operation or else into the feed-discharge mode of operation, the gas (5) is put back into the feed-discharge mode of operation or else into the circulating mode of operation.

25. The method according to Claim 23, **characterized in that**,
during the feed-discharge mode of operation, the gas (5) is put into the circulating mode of operation after a certain time, whereby, immediately before the transition to the circulating mode of operation, the gas quality sensor (18) supplies a first measured value and, immediately after the transition, it supplies a second measured value for the concentration of foreign matter, pollutants or germs, whereby the gas (5) is only put back into the feed-discharge mode of operation once the first measured value is lower than the second measured value or
**in that** the gas (5) in the circulating mode of operation is put into the feed-discharge mode of operation after the certain period of time, whereby, immediately before the transition to the feed-discharge mode of operation, the gas quality sensor (18) supplies a third measured value and, immediately after the transition, it supplies a fourth measured value for the concentration of foreign matter, pollutants or germs, whereby the gas (5) is only returned to the circulating mode of operation once the third measured value is lower than the fourth measured value.

26. The method according to Claim 25, **characterized in that**
the first measured value and the second measured value are compared to each other, and the lower of these two measured values is taken as the limit value or **in that** the third measured value and the fourth measured value are compared to each other and the lower of these two measured values is taken as the limit value, so that the limit value is replaced by a new limit value at certain time intervals.

27. The method according to any of Claims 16 to 26,
**characterized in that** the gas quality sensor signal is employed to increase the flow rate of the gas (5) when the concentration of foreign matter, pollutants or germs in the gas increases, and vice versa.

28. The method according to any of Claims 17 to 27,
**characterized in that** the detrimental influences of humidity, flow rate and gas temperature on the gas quality sensor signal are computationally compensated for.

29. The method according to Claim 28, **characterized in that**,
the concentration of foreign matter, pollutants or germs is shown on a display after the compensation for the detrimental influences of humidity, flow rate and gas temperature.

30. The method according to any of Claims 16 to 29,
**characterized in that** a gas filter (21), for example, an air filter whose capacity to adsorb a test gas added to the gas stream decreases as the gas filter (21) becomes more exhausted, is arranged in the gas stream, whereby
• after the gas stream passes through the gas filter (21), it passes a gas quality sensor (18) that is capable of detecting the test gas and that emits a gas quality sensor signal that is a measure of the concentration of the test gas,
• the test gas is added to the gas stream at a constant concentration for a certain defined period of time before reaching the gas filter (21),
• and, utilizing the fact that the steepness of the rise over time of the gas quality sensor signal diminishes after the beginning of the addition of the test gas and that the steepness of the fall over time of the gas quality sensor signal diminishes after the end of the addition of the test gas as a function of the degree of exhaustion of the gas filter (21), said degree of exhaustion of the gas filter (21) is determined from the steepness of this rise or fall.

31. A device for generating a gas discharge, comprising a gas discharge module (2) with two electrodes (3, 4) located in a gas (5), especially air, and a controllable high-voltage generator (1) that outputs either an alternating voltage or a pulsed direct voltage, whereby the gas discharge module (2) contains a device for generating a plasma for the production of ozone and/or oxygen ions in the air according to the principle of a dielectrically hindered discharge and the electrodes (3, 4) are connected to the high-voltage generator (1) in such a way that the alternating voltage or pulsed direct voltage is present between the electrodes (3, 4), and the gas discharge generates a plasma by means of which the effective surface of the electrodes (3, 4) and thus of the capacitance that exists between them is increased,
**characterized in that**
the device also comprises a controller (6) that compares the actual value of the capacitance that exists between the two electrodes (3, 4), or a value derived from this, with a prescribed setpoint,
whereby, when the actual value of the capacitance that exists between the two electrodes (3, 4) lies below a certain setpoint, the effective value of the high voltage of the high voltage generator (1) is controlled by the controller (6) so as to increase over time,
or when the actual value of the electric capacitance that exists between the two electrodes (3, 4) lies above the setpoint, the effective value of the high voltage of the high voltage generator (1) is controlled by the controller (6) so as to decrease over time.

32. The device according to Claim 31, **characterized in that**
a noise sensor detects the statistical electric noise present in the current that is initiated by the high voltage as a result of the gas discharge and converts it into a second sensor signal, and the actual value of the electric capacitance that exists between the two electrodes (3, 4) is determined from the second sensor signal.

33. The device according to Claim 32, **characterized in that**
the noise sensor comprises:
• a second resistor (11) or an inductor through which the electric current that is initiated by the high voltage passes, and
• a frequency-selective electric filter (12) that functions as a band pass or as a high pass,
whereby the voltage dropping at the second resistor (11) or at the inductor is picked up and, after passing the frequency-selective electric filter (12), serves as the second sensor signal.

34. The device according to any of Claims 31 to 33, **characterized in that**
an acoustic sensor, especially a microphone (12), detects the acoustic noise that is generated by the gas discharge in the gas and converts it into a third sensor signal, and the actual value of the electric capacitance that exists between the two electrodes (3, 4) is determined from the third sensor signal.

35. The device according to any of Claims 31 to 34, **characterized in that**
a radiation sensor (13) detects electromagnetic radiation, e.g. light, X-rays, radio waves, that is generated and emitted by the gas discharge and converts it into a fourth sensor signal, and the actual value of the electric capacitance that exists between the two electrodes (3, 4) is determined from the fourth sensor signal.

36. The device according to any of Claims 31 to 35, **characterized in that**
a phase comparator detects the phase shift between the high voltage and the electric current that is initiated by the high voltage and converts it into a fifth sensor signal, and the actual value of the electric capacitance that exists between the two electrodes (3, 4) is determined from the fifth sensor signal.

37. The device according to any of Claims 31 to 36, **characterized in that**
a current sensor detects the electric current that is initiated by the high voltage and converts it into a first sensor signal, and the actual value of the electric capacitance that exists between the two electrodes is determined from the first sensor signal.

38. The device according to claim 37, **characterized in that**
the current sensor is a first resistor (10) through which the electric current that is initiated by the high voltage passes, whereby the voltage dropping at the first resistor (10) is picked up and serves as the first sensor signal.

39. The device according to Claim 31, **characterized in that**
the waveform of the alternating voltage or of the pulsed direct voltage is sinusoidal or essentially square.

40. The device according to Claim 31, **characterized in that**
the time constant of the regulation is shorter than the half-period of the alternating voltage or of the pulsed direct voltage, so that the waveform of the alternating voltage or of the pulsed direct voltage is influenced by the regulation.

41. The device according to any of Claims 31 to 40, **characterized in that**
the frequency of the alternating voltage or of the pulsed direct voltage is between 15 kHz and 35 kHz.

42. The device according to Claim 31, **characterized in that**
the controller (6) is a PID controller.

43. The device according to any of Claims 31 to 42, **characterized in that**
the gas (5) is air and the setpoint is selected in such a way that ozone and oxygen ions form but no nitrogen oxide NOx forms during the gas discharge.

44. The device according to any of Claims 31 to 33, **characterized in that**
the device for generating a plasma for the production of ozone and/or oxygen ions in the air according to the principle of a dielectrically hindered discharge comprises the following components:
a) a flat, electrically insulating carrier, whose material has a dielectric constant that is at least greater than 30,
b) an electrode (3), namely, the lower electrode (3), made of an electrically conductive material, that is applied onto one of the main surfaces of the carrier, namely, the rear,
c) at least one electric insulating layer made of a dielectric material that is applied onto the other main surface of the carrier, namely, the front, which is exposed to the air, whereby the insulating layer only partially covers the front of the carrier, and the dielectric constant of the carrier and that of the insulating layer are different, whereby the difference between the dielectric constants of the carrier and of the insulating layer or of the partial layers is selected in such a way that the mirror discharge effect occurs,
d) an electrode (4), namely, the upper electrode (4), which is made of an electrically conductive material and which is situated on the insulating layer, only partially covers the insulating layer,
whereby a high voltage from the high voltage generator (1) is applied to the two electrodes (3, 4).

45. The device according to any of the Claims 31 to 44,
**characterized in that** the gas (5) is made to flow by means of a conveying device and a gas quality sensor (18) is arranged in the gas stream in such a way that the gas (5) passes the gas quality sensor (18) before reaching the gas discharge module (2), whereby the gas quality sensor (18) emits a gas quality sensor signal that is a measure of the loading of the gas (5) with foreign matter, pollutants or germs and it is employed to influence the setpoint in such a way that the setpoint is raised or lowered as the loading of the gas (5) with foreign matter, pollutants or germs increases or decreases, respectively.

46. The device according to claim 45, **characterized in that**
a certain setpoint is associated with each concentration of foreign matter, pollutants or germs in the gas (5) in a reversibly unambiguous arrangement.

47. The device according to claim 45, **characterized in that**
the gas quality sensor signal is used to put the gas (5) into a feed-discharge mode of operation in which it is supplied to the gas quality sensor (18) from the outside and, after passing the electrodes (3, 4), it is discharged again to the outside when the concentration of foreign matter, pollutants or germs in the gas (5) has exceeded or fallen below a certain limit value, or else is used to put the gas (5) into a circulating mode of operation in which the gas (5), after passing the electrodes (3, 4), is again fed in a closed circuit to the gas quality sensor (18) when the concentration of foreign matter, pollutants or germs in the gas (5) has exceeded or fallen below a certain limit value.

48. The device according to Claim 47, **characterized in that**,
during the feed-discharge mode of operation, the gas (5) is put into the circulating mode of operation after a certain time, whereby, immediately before the transition to the circulating mode of operation, the gas quality sensor (18) supplies a first measured value and, immediately after the transition, it supplies a second measured value for the concentration of foreign matter, pollutants or germs, whereby the gas (5) is only put back into the feed-discharge mode of operation once the first measured value is lower than the second measured value or
**in that** the gas (5) in the circulating mode of operation is put into the feed-discharge mode of operation after the certain period of time, whereby, immediately before the transition to the feed-discharge mode of operation, the gas quality sensor (18) supplies a third measured value and, immediately after the transition, it supplies a fourth measured value for the concentration of foreign matter, pollutants or germs, whereby the gas (5) is only returned to the circulating mode of operation once the third measured value is lower than the fourth measured value.

49. The device according to claim 48, **characterized in that**
the first measured value and the second measured value are compared to each other and the lower of these two measured values is taken as the limit value or **in that** the third measured value and the fourth measured value are compared to each other and the lower of these two measured values is taken as the limit value, so that the limit value is replaced by a new limit value at certain time intervals.

## Revendications

1. Procédé pour régler la capacité électrique qui existe entre deux électrodes (3, 4) d'un module à décharge électrique dans un gaz (2), ledit module à décharge électrique (2) comprenant un dispositif de génération d'un plasma destiné à la production d'ozone et/ou d'ions d'oxygène dans l'air selon le principe de la décharge à barrière diélectrique, et une haute tension générée par un générateur haute tension réglable (1) servant à déclencher une décharge électrique dans le gaz (5) du module à décharge électrique (2) étant appliquée entre les électrodes (3, 4), ladite haute tension étant ou une tension alternative ou une tension continue pulsée, et la décharge électrique générant un plasma qui agrandit la superficie effective des électrodes (3, 4) et par conséquent la capacité présente entre celles-ci, **caractérisé en ce que**,
lorsque la valeur réelle de la capacité existant entre les deux électrodes (3, 4) est inférieure à une valeur de consigne déterminée, la valeur effective de la haute tension du générateur haute tension (1) est réglée de façon à augmenter dans le temps, ou lorsque la valeur réelle de la capacité électrique existant entre les deux électrodes (3, 4) est supérieure à la valeur de consigne, la valeur effective de la haute tension du générateur haute tension (1) est reglée de façon à baisser dans le temps.

2. Procédé selon la revendication 1, **caractérisé en ce que**
la valeur réelle de la capacité électrique entre les deux électrodes (3, 4) est déterminée ou déduite à partir
- de l'intensité intégrale ou
- de l'intensité d'au moins une fréquence déterminée ou d'au moins une gamme de fréquence déterminée ou
- de la fréquence moyenne ou
- de la répartition des fréquences ou
- de la fréquence de l'intensité maximum
du bruit électrique aléatoire qui, de par la décharge électrique, accompagne le courant électrique mis en marche par la haute tension.

3. Procédé selon la revendication 2, **caractérisé en ce que**
le bruit électrique aléatoire est saisi en faisant passer le courant par une deuxième résistance (R2) ou une inductance, d'où le bruit électrique aléatoire est prélevé via un filtre sélectif (7) qui fait office de filtre passe-bande ou filtre passe-haut.

4. Procédé selon l'une des revendications 1 à 3, **caractérisé en ce que**
la valeur réelle de la capacité électrique entre les deux électrodes (3, 4) est déterminée ou déduite à partir
- de l'intensité intégrale ou
- de l'intensité d'au moins une fréquence déterminée ou d'au moins une gamme de fréquence déterminée ou
- de la fréquence moyenne ou
- de la répartition des fréquences ou
- de la fréquence de l'intensité maximum
du bruit acoustique aléatoire qui, de par la décharge électrique, est généré dans le gaz (5).

5. Procédé selon la revendication 4, **caractérisé en ce que**
le bruit acoustique aléatoire est saisi au moyen d'un transducteur acoustique, en particulier d'un microphone (12).

6. Procédé selon l'une des revendication 1 à 5, **caractérisé en ce que**
la valeur réelle de la capacité électrique entre les deux électrodes (3, 4) est déterminée ou déduite à partir
- de l'intensité intégrale ou
- de l'intensité d'au moins une longueur d'onde ou gamme de longueurs d'onde déterminées ou
- de la longueur d'onde moyenne ou
- de la répartition spectrale des longueurs d'onde ou
- de la longueur d'onde de l'intensité maximum
de rayonnement électromagnétique, tels que la lumière, le rayonnement X, le rayonnement hertzien, généré et émis par la décharge électrique.

7. Procédé selon l'une des revendications 1 à 6, **caractérisé en ce que**
la valeur réelle de la capacité électrique entre les deux électrodes (3, 4) est déterminée ou déduite à partir du déphasage entre la haute tension et le courant électrique mis en mouvement par celle-ci.

8. Procédé selon l'une des revendications 1 à 7, **caractérisé en ce que**
la valeur réelle le la capacité électrique entre les deux électrodes (3, 4) est déterminée ou déduite à partir de la grandeur du courant électrique mis en marche par la haute tension.

9. Procédé selon la revendication 8, **caractérisé en ce que**
le courant électrique est déterminé en le conduisant via une première résistance (R1), où l'on relève la chute de tension, qui sert de mesure pour le courant électrique mis en marche par la haute tension.

10. Procédé selon l'une des revendications 1 à 9, **caractérisé en ce que**
la valeur réelle et la valeur de consigne de la capacité électrique entre les deux électrodes (3, 4) sont comparées à l'aide d'un régulateur (6), tel qu'un régulateur PID, ledit régulateur (6) formant un signal de commande variable dans le temps à partir de la différence entre la valeur réelle et la valeur de consigne ou à partir de l'allure dans le temps de cette différence, lequel signal sert à commander le générateur haute tension (1).

11. Procédé selon l'une des revendications 1 à 10, **caractérisé en ce que**
le gaz (5) est de l'air et la valeur de consigne est choisie de sorte que, lors de la décharge électrique, il se forme de l'ozone et des ions d'oxygène, mais pas d'oxyde d'azote NOₓ.

12. Procédé selon la revendication 1, **caractérisé en ce que**
la tension alternative ou la tension continue pulsée a une forme d'onde sinusoïdale ou sensiblement rectangulaire.

13. Procédé selon la revendication 1, **caractérisé en ce que**
la constante de temps de la régulation est plus courte que la demi-période de la tension alternative ou de la tension continue pulsée, de sorte que la forme d'onde de la tension alternative ou de la tension continue pulsée est influencée par la régulation.

14. Procédé selon l'une des revendications 1 à 13, **caractérisé en ce que**
la fréquence de la tension alternative ou de la tension continue pulsée est comprise entre 15 kHz et 35 kHz.

15. Procédé selon l'une des revendications 1 à 4, **caractérisé en ce que**
le dispositif de génération d'un plasma destiné à la production d'ozone et/ou d'ions d'oxygène dans l'air d'après le principe de la décharge à barrière diélectrique comprend les composants suivants :
a) un support plat isolant électriquement dont le matériau présente une constante diélectrique au moins supérieure à 30,
b) une électrode (3), électrode inférieure (3), disposée sur l'une des surfaces principales du support, face arrière, en un matériau électroconducteur,
c) au moins une couche d'isolation électrique consistant en un matériau diélectrique et appliquée sur l'autre surface principale du support, face avant, laquelle est exposée à l'air, ladite couche d'isolation ne recouvrant la face avant que partiellement et la constante diélectrique du support et celle de la couche d'isolation étant différentes, la différence entre la constante diélectrique du support et celle de la couche d'isolation ou des couches partielles étant choisie de façon à ce qu'on obtienne l'effet des décharges symétriques,
d) une électrode (4), électrode supérieure, disposée sur la couche d'isolation, consistant en un matériau électroconducteur et ne recouvrant la couche d'isolation que partiellement, une haute tension du générateur haute tension (1) étant appliquée aux deux électrodes (3, 4).

16. Procédé selon l'une des revendications 1 à 15, **caractérisé en ce que**
le gaz (5) est transporté par un dispositif de transport, tel qu'un ventilateur (20), de sorte qu'il passe à travers le module à décharge électrique (2).

17. Procédé selon la revendication 16, **caractérisé en ce que**
un capteur de qualité de gaz (18) est disposé dans le flux de gaz de telle manière que le gaz (5) passe par le capteur de qualité de gaz (18) avant d'atteindre le module à décharge électrique (2), ledit capteur de qualité de gaz (18) émettant un signal de capteur de qualité de gaz qui constitue une mesure de la contamination du gaz (5) par des impuretés, des substances nocives ou des germes et qui est utilisé pour influer sur la valeur de consigne de sorte que la valeur de consigne augmente ou baisse au fur et à mesure que la contamination du gaz (5) par des impuretés, des substances nocives ou des germes augmente ou diminue.

18. Procédé selon la revendication 1, **caractérisé en ce que**
les électrodes (3, 4) se trouvent dans un gaz (5) qui s'écoule, en particulier de l'air, et que, pour régler la tension de sortie du générateur haute tension (1), un régulateur (6, 16) compare la valeur de tension réelle avec une valeur de tension de consigne et que, lorsque la valeur de tension réelle est inférieure à la valeur de tension de consigne, la haute tension est réglée de façon à augmenter dans le temps ou bien, lorsque la valeur de tension réelle est supérieure à la valeur de tension de consigne, la haute tension est réglée de façon à baisser dans le temps, un capteur de qualité de gaz (18) étant disposé dans le flux de gaz de telle manière que le gaz (5) passe par le capteur de qualité de gaz (18) avant d'atteindre les électrodes (3, 4), ledit capteur de qualité de gaz (18) émettant un signal de capteur de qualité de gaz qui constitue une mesure de la contamination du gaz (5) par des impuretés, des substances nocives ou des germes et qui est utilisé pour influer sur la valeur de tension de consigne de sorte que la valeur de tension de consigne augmente ou baisse au fur et à mesure que la contamination du gaz (5) par des impuretés, des substances nocives ou des germes augmente ou diminue.

19. Procédé selon la revendication 1, **caractérisé en ce que**
les électrodes (3, 4) se trouvent dans un gaz (5) qui s'écoule, en particulier de l'air, et que, pour régler la puissance d'ionisation de la décharge électrique, la haute tension est appliquée entre les électrodes (3, 4) via une troisième résistance (R3) et la valeur de puissance d'ionisation réelle est déterminée à partir de la valeur de la haute tension et de la tension en chute à la troisième résistance (R3) et qu'un régulateur (6, 16) compare la valeur de puissane d'ionisation réelle avec une valeur de puissance d'ionisation de consigne et, lorsque la valeur de puissance d'ionisation réelle est inférieure à la valeur de puissance d'ionisation de consigne, la haute tension est réglée de façon à augmenter dans le temps ou bien, lorsque la valeur de puissance d'ionisation réelle est supérieure à la valeur de puissance d'ionisation de consigne, la haute tension est réglée de façon à baisser dans le temps, un capteur de qualité de gaz (18) étant disposé dans le flux de gaz de telle manière que le gaz (5) passe par le capteur de qualité de gaz (18) avant d'atteindre les électrodes (3, 4), ledit capteur de qualité de gaz (18) émettant un signal de capteur de qualité de gaz qui constitue une mesure de la contamination du gaz (5) par des impuretés, des substances nocives ou des germes et qui est utilisé pour influer sur la valeur de tension de consigne de sorte que la valeur de puissance d'ionisation de consigne augmente ou baisse au fur et à mesure que la contamination du gaz (5) par des impuretés, des substances nocives ou des germes augmente ou diminue.

20. Procédé selon l'une des revendications 17 à 19, **caractérisé en ce que**
le capteur de qualité de gaz (18) est un capteur d' odeurs ou de germes.

21. Procédé selon l'une des revendications 17 à 19, **caractérisé en ce que**
le capteur de qualité de gaz (18) est un capteur d'impuretés ou de substances nocives gazeuses ou sous forme de particules.

22. Procédé selon l'une des revendications 17 à 19, **caractérisé en ce que**
le gaz (5) est de l'air et le capteur de qualité de gaz (18) est un capteur d'impuretés ou de substances nocives gazeuses ou sous forme de particules contenues dans l'air et oxydables.

23. Procédé selon l'une des revendications 17 à 22, **caractérisé en ce que**
le signal du capteur de qualité de gaz est utilisé pour mettre le gaz (5) dans un régime d'amenée/de sortie, dans lequel il est amené au capteur de qualité de gaz (18) de l'extérieur et, après être passé par les électrodes (3, 4), réacheminé vers l'extérieur, lorsque la concentration d'impuretés, de substances nocives ou de germes dans le gaz (5) est supérieure ou inférieure à une valeur limite déterminée, ou pour mettre le gaz (5) dans un régime de circulation, dans lequel de gaz, après être passé par les électrodes (3, 4), est reconduit en circuit fermé au capteur de qualité de gaz (18), lorsque la concentration d'impuretés, de substances nocives ou de germes dans le gaz (5) est inférieure ou supérieure à une valeur limite déterminée.

24. Procédé selon la revendication 23, **caractérisé en ce que**
lorsque la concentration d'impuretés, de substances nocives ou de germes dans le gaz (5) continue à augmenter après que le gaz (5) ait été mis en régime de circulation ou en régime d'amenée/de sortie, le gaz (5) est remis respectivement en régime d'amenée/de sortie ou en régime de circulation.

25. Procédé selon la revendication 23, **caractérisé en ce que**
lorsque le gaz (5) se trouve en régime d'amenée/de sortie, il est remis en régime de circulation après un intervalle de temps déterminé, le capteur de qualité de gaz (18) fournissant une première valeur de mesure de la concentration d'impuretés, de substances nocives ou de germes immédiatement avant et une deuxième valeur de mesure immédiatement après le passage en régime de circulation, le gaz (5) n'étant remis en régime d'amenée/de sortie qu'au cas
où la première valeur de mesure est inférieure à la deuxième valeur de mesure, ou
**en ce que**, lorsque le gaz (5) se trouve en régime de circulation, il est remis en régime d'amenée/de sortie après ledit intervalle de temps déterminé, le capteur de qualité de gaz (18) fournissant une troisième valeur de mesure de la concentration d'impuretés, de substances nocives ou de germes immédiatement avant et une quatrième valeur de mesure immédiatement après le passage en régime d'amenée/de sortie, le gaz (5) n'étant remis en régime de circulation qu'au cas où la troisième valeur de mesure est inférieure à la quatrième valeur de mesure.

26. Procédé selon la revendication 25, **caractérisé en ce que**
la première et la deuxième valeur de mesure sont comparées entre elles et la valeur la moins élevée de ces deux valeurs de mesure est reprise comme valeur limite, ou **en ce que** la troisième et la quatrième valeur de mesure sont comparées entre elles et la valeur la moins élevée de ces deux valeurs de mesure est reprise comme valeur limite, de sorte que la valeur limite est remplacée par une nouvelle valeur limite dans lesdits intervalles de temps déterminés.

27. Procédé selon l'une des revendications 16 à 26, **caractérisé en ce que**
le signal du capteur de qualité de gaz est utilisé à augmenter la vitesse d'écoulement du gaz (5) au fur et à mesure que la concentration d'impuretés, de substances nocives ou de germes dans le gaz augmente et vice versa.

28. Procédé selon l'une des revendications 17 à 27, **caractérisé en ce que**
les effets perturbateurs de l'humidité de l'air, de la vitesse d'écoulement et de la température du gaz sur le signal du capteur de qualité de gaz sont compensés par calcul.

29. Procédé selon la revendication 28, **caractérisé en ce que**,
après compensation des effets perturbateurs de l'humidité de l'air, de la vitesse d'écoulement et de la température du gaz, la concentration d'impuretés, de substances nocives ou de germes est affichée sur un visuel.

30. Procédé selon l'une des revendications 16 à 29, **caractérisé en ce que**
un filtre à gaz (21), tel qu'un filtre à air, est disposé dans le flux de gaz, dont la capacité d'absorber un gaz témoin ajouté au flux de gaz diminue au fur et à mesure de l'épuisement du filtre à gaz (21),
- le flux de gaz, après avoir traversé le filtre à gaz (21), passe par un capteur de qualité de gaz (18) qui est capable de détecter le gaz témoin et émet un signal de capteur de qualité de gaz constituant une mesure de la concentration du gaz témoin,
- le gaz témoin étant ajouté au flux de gaz pendant une période de temps définie et selon une concentration constante avant que ledit flux de gaz n'ait atteint le filtre à gaz (21),
- et, en mettant à profit le fait que la pente de la montée du signal du capteur de qualité de gaz dans le temps après le début, et celle de la descente du signal du capteur de qualité de gaz dans le temps à la fin de l'ajout du gaz témoin diminuent, le degré d'épuisement du filtre à gaz (21) est déterminé à partir de la pente de cette montée ou de cette descente.

31. Dispositif destiné à générer une décharge électrique dans un gaz, comprenant un module à décharge électrique (2) avec deux électrodes (3, 4) disposées dans un gaz (5), notamment dans l'air, et un générateur haute tension réglable (1) générant ou une tension alternative ou une tension constante pulsée, le module à décharge électrique (2) comprenant un dispositif de génération d'un plasma destiné à la production d'ozone et/ou d'ions d'oxygène dans l'air d'après le principe de la décharge à barrière diélectrique, et les électrodes (3, 4) étant reliées au générateur haute tension (1) de sorte que la tension alternative ou la tension constante pulsée est appliquée entre les électrodes (3, 4) et la décharge électrique générant un plasma qui agrandit la superficie effective des électrodes (3, 4) et par conséquent la capacité existant entre celles-ci, **caractérisé en ce que**
ledit dispositif comprend en outre un régulateur (6) qui compare la valeur réelle de la capacité entre les deux électrodes (3, 4) ou une valeur déduite de celle-ci avec une valeur de consigne prédéfinissable,
la valeur effective de la haute tension du générateur haute tension (1) étant réglée de façon à augmenter dans le temps lorsque la valeur réelle de la capacité présente entre les deux électrodes (3, 4) est inférieure à une valeur de consigne déterminée,
ou la valeur effective de la haute tension du générateur haute tension (1) étant reglée de façon à baisser dans le temps lorsque la valeur réelle de la capacité électrique présente entre les deux électrodes (3, 4) est supérieure à la valeur de consigne.

32. Dispositif selon la revendication 31, **caractérisé en ce que**
un capteur de bruit saisit le bruit électrique aléatoire qui, de par la décharge électrique, accompagne le courant électrique mis en marche par la haute tension, et le transforme en un deuxième signal de capteur et que la valeur réelle de la capacité électrique entre les deux électrodes (3, 4) est déterminée à partir dudit deuxième signal de capteur.

33. Dispositif selon la revendication 32, **caractérisé en ce que**
le capteur de bruit comprend :
- une deuxième résistance (11) ou une inductance par laquelle passe le courant électrique mis en marche par la haute tension et
- un filtre électrique sélectif (12) faisant office de filtre passe-bande ou filtre passe-haut,
la tension en chute à la deuxième résistance (11) ou à l'inductance étant prélevée et, après le passage à travers le filtre électrique sélectif (12), sert de deuxième signal de capteur.

34. Dispositif selon l'une des revendications 31 à 33, **caractérisé en ce que**
un capteur acoustique, notamment un microphone (12), saisit le bruit acoustique généré par la décharge électrique dans le gaz et le transforme en un troisième signal de capteur, et que la valeur réelle de la capacité électrique entre les deux électrodes (3, 4) est déterminée à partir du troisième signal de capteur.

35. Dispositif selon l'une des revendications 31 à 34, **caractérisé en ce que**
un capteur de rayonnement (13) saisit le rayonnement électromagnétique tel que la lumière, le rayonnement X ou le rayonnement hertzien généré et émis par la décharge électrique et le transforme en un quatrième signal de capteur, et que la valeur réelle de la capacité électrique entre les deux électrodes (3, 4) est déterminée à partir du quatrième signal de capteur.

36. Dispositif selon l'une des revendications 31 à 35, **caractérisé en ce que**
un comparateur de phase saisit le déphasage entre la haute tension et le courant électrique mis en marche par celle-ci et le transforme en un cinquième signal de capteur, et que la valeur réelle de la capacité électrique entre les deux électrodes (3, 4) est déterminée à partir du cinquième signal de capteur.

37. Dispositif selon l'une des revendications 31 à 36, **caracterisé en ce que**
un capteur de courant saisit le courant électrique mis en marche par la haute tension et le transforme en un premier signal de capteur, et que la valeur réelle de la capacité électrique entre les deux électrodes (3, 4) est déterminée à partir du premier signal de capteur

38. Dispositif selon la revendication 37, **caractérisé en ce que**
le capteur de courant est une première résistance (10) par laquelle passe le courant électrique mis en marche par la haute tension, la tension en chute à la première résistance (10) étant prélevée et servant de premier signal de capteur.

39. Dispositif selon la revendication 31, **caractérisé en ce que**
la tension alternative ou la tension constante pulsée a une forme d'onde sinusoïdale ou sensiblement rectangulaire.

40. Dispositif selon la revendication 31, **caractérisé en ce que**
la constante de temps de la régulation est plus courte que la demi-période de la tension alternative ou de la tension continue pulsée, de sorte que la forme d'onde de la tension alternative ou de la tension continue pulsée est influencée par la régulation.

41. Dispositif selon l'une des revendications 31 à 40, **caractérisé en ce que**
la fréquence de la tension alternative ou de la tension continue pulsée est comprise entre 15 kHz et 35 kHz.

42. Dispositif selon la revendication 31, **caractérisé en ce que**
le régulateur (6) est un régulateur PID.

43. Dispositif selon l'une des revendications 31 à 42, **caractérisé en ce que**
le gaz (5) est de l'air et la valeur de consigne est choisie de sorte que, lors de la décharge électrique, il se forme de l'ozone et des ions d'oxygène, mais pas d'oxyde d'azote.

44. Dispositif selon l'une des revendications 31 à 33, **caractérisé en ce que**
le dispositif de génération d'un plasma destiné à la production d'ozone et/ou d'ions d'oxygène dans l'air d'après le principe de la décharge à barrière diélectrique comprend les composants suivants :
a) un support plat isolant électriquement dont le matériau présente une constante diélectrique au moins supérieure à 30,
b) une électrode (3), électrode inférieure (3), disposée sur l'une des surfaces principales du support, face arrière, en un matériau électroconducteur,
c) au moins une couche d'isolation électrique consistant en un matériau diélectrique et appliquée sur l'autre surface principale du support, face avant, laquelle est exposée à l'air, ladite couche d'isolation ne recouvrant la face avant que partiellement et la constante diélectrique du support et celle de la couche d'isolation étant différentes, la différence entre la constante diélectrique du support et celle de la couche d'isolation ou des couches partielles étant choisie de façon à ce qu'on obtienne l'effet des décharges symétriques,
d) une électrode (4), électrode supérieure (4), disposée sur la couche d'isolation, consistant en un matériau électroconducteur et ne recouvrant la couche d'isolation que partiellement,
une haute tension du générateur haute tension (1) étant appliquée aux deux électrodes (3, 4).

45. Dispositif selon l'une des revendications 31 à 44, **caractérisé en ce que**
le gaz (5) est mis en écoulement par un moyen de transport et un capteur de qualité de gaz (18) est disposé dans le flux de gaz de telle manière que le gaz (5) passe par le capteur de qualité de gaz (18) avant d'atteindre le module à décharge électrique (2), ledit capteur de qualité de gaz (18) émettant un signal de capteur de qualité de gaz qui constitue une mesure de la contamination du gaz (5) par des impuretés, des substances nocives ou des germes et qui est utilisé pour influer sur la valeur de consigne de sorte que la valeur de consigne augmente ou baisse au fur et à mesure que la contamination du gaz (5) par des impuretés, des substances nocives ou des germes augmente ou diminue.

46. Dispositif selon la revendication 45, **caractérisé en ce que**
une valeur de consigne déterminée est assignée biunivoquement à chaque concentration d'impuretés, de substances nocives ou de germes dans le gaz (5).

47. Dispositif selon la revendication 45, **caractérisé en ce que**
le signal du capteur de qualité de gaz est utilisé pour mettre le gaz (5) dans un régime d'amenée/de sortie, dans lequel il est amené au capteur de qualité de gaz (18) de l'extérieur et, après être passé par les électrodes (3, 4), réacheminé vers l'extérieur, lorsque la concentration d'impuretés, de substances nocives
ou de germes dans le gaz (5) est supérieure ou inférieure à une valeur limite déterminée, ou pour mettre le gaz (5) dans un régime de circulation, dans lequel de gaz, après être passé par les électrodes (3, 4), est reconduit en circuit fermé au capteur de qualité de gaz (18), lorsque la concentration d'impuretés, de substances nocives ou de germes dans le gaz (5) est inférieure ou supérieure à une valeur limite déterminée.

48. Dispositif selon la revendication 47, **caractérisé en ce que**
lorsque le gaz (5) se trouve en régime d'amenée/de sortie, il est remis en régime de circulation après un intervalle de temps déterminé, le capteur de qualité de gaz (18) fournissant une première valeur de mesure de la concentration d'impuretés, de substances nocives ou de germes immédiatement avant et une deuxième valeur de mesure immédiatement après le passage en régime de circulation, le gaz (5) n'étant remis en régime d'amenée/de sortie qu'au cas
où la première valeur de mesure est inférieure à la deuxième valeur de mesure, ou
**en ce que**, lorsque le gaz (5) se trouve en régime de circulation, il est remis en régime d'amenée/de sortie après ledit intervalle de temps déterminé, le capteur de qualité de gaz (18) fournissant une troisième valeur de mesure de la concentration d'impuretés, de substances nocives ou de germes immédiatement avant et une quatrième valeur de mesure immédiatement après le passage en régime d'amenée/de sortie, le gaz (5) n'étant remis en régime de circulation qu'au cas où la troisième valeur de mesure est inférieure à la quatrième valeur de mesure.

49. Dispositif selon la revendication 48, **caractérisé en ce que**
la première et la deuxième valeur de mesure sont comparées entre elles et la valeur la moins élevée de ces deux valeurs de mesure est reprise comme valeur limite, ou **en ce que** la troisième et la quatrième valeur de mesure sont comparées entre elles et la valeur la moins élevée de ces deux valeurs de mesure est reprise omme valeur limite, de sorte que la valeur limite est remplacée par une nouvelle valeur limite dans lesdits intervalles de temps déterminés.
